# EUROPÄISCHE PATENTSCHRIFT

(11) **EP 4 344 712 B1**
(45) Veröffentlichungstag und Bekanntmachung des Hinweises auf die Patenterteilung: **13.05.2026**
(21) Anmeldenummer: 24158388.9
(22) Anmeldetag: 24.10.2017
(51) Int. Cl.: A61M 1/06

(54) **BRUSTPUMPE**
BREAST PUMP
POMPE TIRE-LAIT

(43) Veröffentlichungstag der Anmeldung: 03.04.2024
(62) Teilanmeldung aus: 17787438.5
(73) Patentinhaber: Medela Holding AG, 6340 Baar (CH)
(72) Erfinder: Schlienger, André, 8933 Maschwanden (CH); Höner, Sebastian, 8800 Thalwil (CH); Rigert, Mario, 6033 Buchrain (CH); Felber, Armin, 6003 Luzern (CH); Steiner, Marco, 6330 Cham (CH)
(74) Vertreter: Grünecker Patent- und Rechtsanwälte PartG mbB

(56) Entgegenhaltungen:
- EP-A1- 3 058 967
- WO-A1-2016/014469
- WO-A1-2016/014494

## Beschreibung

### TECHNISCHES GEBIET

Die vorliegende Erfindung betrifft eine Brustpumpe zum Abpumpen von menschlicher Muttermilch.

### STAND DER TECHNIK

Vorrichtungen zum Abpumpen von menschlicher Muttermilch sind hinlänglich bekannt. Grundsätzlich gibt es zwei verschiedene Typen: die ersten werden manuell bedient, d.h. der für das Abpumpen notwendige Unterdruck wird durch manuelle Betätigung der Vakuumpumpe erzeugt. Bei den zweiten Typen ist die Vakuumpumpe elektrisch betrieben.

Diese Vakuumpumpen sind entweder direkt oder über eine Vakuumleitung mit einer Brusthaube verbunden. Die Brusthaube weist einen Innenraum zur Aufnahme der Mutterbrust bzw. deren Brustwarze auf. Der Unterdruck wird über eine erste Leitung in den Innenraum übertragen, die abgepumpte Milch fliesst über eine zweite Leitung in einen Milchsammelbehälter.

WO 2016/014494 A1 und WO 2016/014469 A1 beschreiben eine kompakte Brustpumpe, bei welcher abgepumpte Milch unter positivem Druck in einen Milchsammelbehälter transportiert wird. Hierfür wird beispielsweise eine Milchleitung zyklisch gequetscht.

WO 2011/035447 A1 offenbart eine Brustpumpe, bei welcher die Vakuumleitung gleichzeitig die Milchleitung bildet. Die Pumpkammer, in welcher der Unterdruck erzeugt wird, füllt sich während des Abpumpens mit Milch, so dass das Pumpsystem von einem pneumatischen System in ein hydraulisches System wechselt und die bereits abgepumpte

Milch zum Arbeitsfluid wird. Damit sich die Pumpkammer mit Milch füllen kann, ist am Ausgang der Pumpkammer, welcher zum Milchsammelbehälter fuhrt, ein Ventil angeordnet. Dieses Ventil öffnet sich erst bei einem bestimmten Druck innerhalb der Pumpkammer, d.h. wenn die Pumpkammer ausreichend mit Milch gefüllt ist.

Diese kombinierte pneumatische und hydraulische Pumpe weist mehrere Vorteile auf. Das System weist kein oder fast kein Totvolumen auf. Das Pumpaggregat kann minimiert werden, da nach Füllung des Systems mit Milch nur mehr eine geringe Pumpleistung notwendig ist, um weitere Milch abzupumpen. Insbesondere lassen sich Batterien mit geringerer Kapazität verwenden als bei pneumatischen Vakuumpumpen. Die Vakuumpumpe lässt sich somit kostengünstiger und kleiner ausbilden. Die Brustpumpe eignet sich insbesondere für sogenannte "hands-free" Anwendungen, bei welchen die Brustpumpe am Körper getragen und keine Hände zum Festhalten der Brusthaube benötigt werden. Aufgrund des minimierten Totvolumens lässt sich zudem der angelegte Druck leichter kontrollieren. Dadurch lassen sich für jede Mutter individuell optimierte Pumpparameter wählen.

In WO 2013/049944 A2 ist ebenfalls eine Brustpumpe beschrieben, welche von einem pneumatischen in ein hydraulisches Pumpsystem wechselt. In der Pumpkammer ist ein Drucksensor vorhanden, welcher eine an einer flexiblen Membran angebrachte Fahne umfasst.

Eine weitere relevante Brustpumpe aus dem Stand der Technik ist beispielsweise in EP3058967 A1 offenbart.

### DARSTELLUNG DER ERFINDUNG

Es ist deshalb eine Aufgabe der Erfindung, die oben genannte Brustpumpe zu optimieren.

Diese Aufgabe löst eine Brustpumpe gemäss Anspruch 1.

In einer bevorzugten Ausführungsform weist die offenbarte Brustpumpe zum Abpumpen von menschlicher Muttermilch weist ein Pumpengehäuse mit einer Antriebseinheit, eine Pumpkammer mit einer von der Antriebseinheit angetriebenen flexiblen Pumpmembran zur Erzeugung eines Vakuums und eine Brusthaube mit einem Innenraum zur Aufnahme einer Mutterbrust auf. Der Innenraum ist mittels der Pumpkammer mit einem sich zyklisch ändernden Unterdruck beaufschlagbar, wobei ein Milchkanal vom Innenraum durch eine erste Einlassöffnung als erste Durchgangsöffnung in die Pumpkammer führt, wobei abgepumpte Milch vom Innenraum der Brusthaube durch den Milchkanal in die Pumpkammer fliesst, und wobei die Pumpkammer eine Auslassöffnung aufweist, durch welche die abgepumpte Milch von der Pumpkammer in einen Milchsammelbehälter fliesst. Ein mehrteiliges Hygienemodul ist vorhanden, wobei das Hygienemodul einen ersten Teil mit einer Durchflussöffnung zur Verbindung mit dem Innenraum, einen Ventilkörper mit mindestens einem Ventil und einen zweiten Teil mit einer steifen ersten Schale zur Bildung der Pumpkammer aufweist, wobei der Ventilkörper zwischen dem ersten und zweiten Teil angeordnet ist und wobei das Hygienemodul als Ganzes mit dem Pumpengehäuse der Brustpumpe dichtend und lösbar verbindbar ist.

Dieses Hygienemodul ist kompakt aufgebaut. Es lässt sich einfach montieren und auseinander nehmen, so dass sich die relevanten Teile der Brustpumpe einfach reinigen lassen. Die Verwendung eines Ventilkörpers im Hygienemodul erleichtert die Herstellung, den Zusammenbau und die Reinigung zusätzlich.

Vorzugsweise weist die steife erste Schale einen Milchpfad auf, welcher von der Pumpkammer in einen Milchsammelbehälter fühlt und welcher mindestens über einen Teilbereich senkrecht zu einer Antriebsachse der Pumpmembran verläuft. Vorzugsweise verläuft der Milchpfad in einer Ebene senkrecht zur Antriebsachse, wobei sich der Milchpfad in dieser Ebene über mindestens die Hälfte der Höhe der Pumpkammer, vorzugsweise über die gesamte Höhe der Pumpkammer erstreckt. Der Verlauf dieses Milchpfades ermöglicht es, die Pumpkammer mit abgepumpter Milch mindestens teilweise, vorzugsweise vollständig zu füllen, so dass während des Abpumpens von einem pneumatischen Pumpsystem auf ein gemischtes oder sogar vollständig hydraulisch funktionierendes Pumpsystem gewechselt wird.

Vorzugsweise weist der Ventilkörper mindestens ein Durchflussventil und ein Belüftungsventil auf. Vorzugsweise ist der Ventilkörper einstückig aus einem flexiblen

Material gebildet. Dies erleichtert sowohl die Herstellung, wie auch den Zusammenbau und die Reinigung. Vorzugsweise ist der erste Teil ein Bestandteil der Brusthaube. Dadurch lässt sich die Brustpumpe kompakt ausbilden und das Hygienemodul lässt sich auf einfache Art und Weise an der Brustpumpe befestigen, ohne dass zusätzlich noch die Brusthaube montiert werden muss.

Vorzugsweise weist die Brusthaube einen Flansch zur lösbaren Verbindung mit dem Pumpengehäuse auf. Die Verbindung mit dem Pumpengehäuse lässt sich auf besonders einfache Art und Weise herstellen, wenn sie form- und kraftschlüssig, vorzugsweise ein Bajonettverschluss, ist.

Eine Brustpumpe gemäß der vorliegenden Erfindung weist die in Anspruch 1 definierten technischen Merkmale auf.

Die erfindungsgemässe Brustpumpe zum Abpumpen von menschlicher Muttermilch weist eine Brusthaube mit einem Innenraum zur Aufnahme einer Mutterbrust und eine Pumpkammer auf, wobei der Innenraum mittels der Pumpkammer mit einem sich zyklisch ändernden Unterdruck beaufschlagbar ist. Ein Milchkanal führt vom Innenraum durch eine erste Einlassöffnung in die Pumpkammer, durch welchen abgepumpte Milch vom Innenraum der Brusthaube in die Pumpkammer fliesst. Die Pumpkammer weist eine Auslassöffnung auf, durch welche die abgepumpte Milch von der Pumpkammer in einen Milchsammelbehälter fliesst. Die Auslassöffnung ist in vertikaler Gebrauchslage der Brustpumpe mindestens zu Beginn eines Abpumpvorgangs in einem oberen Bereich der Pumpkammer und/oder oberhalb der Einlassöffnung angeordnet.

Dadurch lässt sich die Pumpkammer annähernd vollständig mit Milch füllen und das System kann innerhalb kürzester Zeit von einem pneumatischen in ein hydraulisches oder gemischt pneumatisch/hydraulisches System übergeführt werden. Dieselbe Antriebsleistung für die Pumpmittel, z.B. der Pumpmembran, führt zu einer grösseren Pumpleistung, d.h. zu einem betragsmässig höheren Unterdruck in der Pumpkammer und somit im Innenraum der Brusthaube. Es lassen sich Batterien mit geringerer Kapazität verwenden. Dies vermindert die Herstellungskosten, erhöht die Lebensdauer der Pumpe insgesamt
und minimiert die Grösse der Antriebseinheit.

Vorzugsweise bildet der Milchkanal einen Vakuumkanal zur Beaufschlagung des Innenraums der Brusthaube mit dem sich zyklisch ändernden Unterdruck. D.h. es wird über dieselbe Leitung bzw. über denselben Kanal zuerst das Vakuum angelegt und anschliessend die Milch abgesaugt. Dieselbe Leitung bzw. derselbe Kanal wird für beide Arbeitsfluide, d.h. Luft und Milch, verwendet.

Je nach Ausführungsform ist die Pumpe eine Kolbenpumpe, eine Membranpumpe oder eine andere Art Vakuumpumpe. Erfindungsgemäß weist die Pumpkammer jedoch eine steife erste Schale und eine flexible Pumpmembran auf, wobei die Pumpmembran dichtend an der steifen ersten Schale anliegt und wobei die Pumpmembran angetrieben ist und durch deren Bewegung relativ zur steifen ersten Schale ein Unterdruck in der Pumpkammer erzeugbar ist. Vorzugsweise ist zwischen der steifen ersten Schale und der flexiblen Pumpmembran eine flexible Medientrennmembran angeordnet, so dass die Pumpmembran vor einem Kontakt mit abgepumpter Milch geschützt ist. Milch und Luft, welche in der Brusthaube vorliegen, gelangen nur bis zur Medientrennmembran. Die Pumpmembran und die übrigen Teile der Brustpumpe sind somit vor einer Kontamination geschützt.

Die Medientrennmembran lässt sich auf einfache Art und Weise von der Pumpmembran trennen. Sie ist vorzugsweise Teil des bereits erwähnten Hygienemoduls. Die Medientrennmembran lässt sich vorzugsweise von den übrigen Teilen des Hygienemoduls trennen und so auf einfache Art und Weise reinigen. Auch die übrigen Teile des Hygienemoduls lassen sich soweit notwendig voneinander trennen, so dass sie gereinigt und anschliessend auf einfache Art und Weise und vorzugsweise ohne Werkzeuge wieder zusammen gesetzt und erneut verwendet werden können.

Vorzugsweise weist die steife erste Schale eine Auslassrinne auf, welche sich von der Auslassöffnung der Pumpkammer zu einem Ausgang erstreckt, wobei die Auslassrinne mindestens über einen Teilbereich in einer Richtung senkrecht zur Bewegungsrichtung der Pumpmembran verläuft. Vorzugsweise verläuft sie in einer Ebene senkrecht zu dieser Bewegungsrichtung, wobei sich die Rinne vorzugsweise über mindestens die Hälfte der

Höhe der Pumpkammer, vorzugsweise über die gesamte Pumpkammer erstreckt.

**In** anderen Ausführungsformen ist die Rinne teilweise oder ganz als geschlossener Kanal ausgebildet. Vorzugsweise verschliesst jedoch die Pumpmembran, oder, falls vorhanden, die Medientrennmembran die Auslassrinne der steifen ersten Schale zu einem bis auf die Auslassöffnung und den Ausgang geschlossenen Kanal.

Die Rinne erstreckt sich vorzugsweise neben der Pumpkammer und nicht innerhalb derselben.

In einer bevorzugten Ausführungsform weist die Brusthaube eine erste Durchlassöffnung auf, welche Teil des Milchkanals bildet, wobei die Durchlassöffnung in Gebrauchslage der Brusthaube mindestens zu Beginn des Abpumpvorgangs in einem oberen Bereich des Innenraums angeordnet ist, so dass der Innenraum unterhalb der ersten Durchlassöffnung mit abgepumpter Milch befüllbar ist.

Dadurch lässt sich die Brusthaube zu Beginn des Abpumpvorgangs mit Milch teilweise oder ganz füllen. Dies verkürzt die Zeit bis zum Wechsel auf das hydraulische Pumpsystem nochmals massiv. Üblicherweise wird zuerst der Innenraum der Brusthaube gefüllt und anschliessend die Pumpkammer, bevor die Milch erstmals in den Milchsammelbehälter gelangt.

Vorzugsweise ist der Milchkanal mittels eines ersten Ventils verschliessbar, um am Ende des Abpumpvorgangs die abgepumpte Milch aus der Pumpkammer zu entfernen. Dieses Ventil ist vorzugsweise dasselbe Ventil, welches sich beim Abpumpen der Milch selbsttätig öffnet und schliesst. Für die Entleerung wird es nun permanent geschlossen. Die Schliessung erfolgt je nach Ausführungsform manuell oder automatisch, z.B. mittels der elektronischen Steuerung der Brustpumpe. Ist nachfolgend ein Ventil angeordnet, so kann das erste Ventil während des Abpumpvorgangs auch stets geöffnet sein.

Vorzugsweise ist ein Entleerungskanal zwischen dem Innenraum der Brusthaube und der Pumpkammer vorhanden, welcher getrennt vom Milchkanal verläuft, wobei der Entleerungskanal mittels eines zweiten Ventils verschliessbar ist. Der Entleerungskanal ist
während des Abpumpvorgangs geschlossen und ist am Ende des Abpumpvorgangs geöffnet. Auch dies erfolgt je nach Ausführungsform manuell oder automatisch. Das zweite Ventil funktioniert vorzugsweise gleich oder ähnlich wie das erste Ventil. Es ist vorzugsweise identisch ausgebildet. Es öffnet und schliesst sich während des Pumpvorgangs beim Entleeren der Brusthaube gleich wie das erste Ventil während des Abpumpens der Muttermilch aus der Mutterbrust. Ist nachfolgend ein Ventil angeordnet, so kann das zweite Ventil während des Entleerungsvorgangs auch stets geöffnet sein.

Vorzugsweise ist zudem ein Belüftungsventil zur Belüftung des Innenraums der Brusthaube vorhanden. Dieses ist ebenfalls vorzugsweise während des Abpumpvorgangs stets geschlossen und wird manuell oder automatisch geöffnet, um das Entleeren der Brusthaube zu erleichtern.

Vorzugsweise ist das Belüftungsventil gemeinsam mit dem zweiten Ventil verschliessbar und es lässt sich gemeinsam mit diesem öffnen.

In einer bevorzugten Ausführungsform ist ein drittes Ventil vorhanden, welches bei Druckanstieg des sich zyklisch ändernden Unterdrucks im Innenraum der Brusthaube einen Basisunterdruck beibehält. Der Druck im Innenraum der Brusthaube liegt somit während des gesamten Abpumpvorgangs und vorzugsweise auch während der Entleerung unter dem Atmosphärendruck. Dies vermindert die Belastung der Brustwarze und wirkt sich positiv auf den Milchfluss aus der Mutterbrust aus.

Vorzugsweise ist im Milchkanal ein viertes Ventil vorhanden, welches sich nach Massgabe des angelegten Unterdrucks öffnet und schliesst. Dieses vierte Ventil ist in Abpumprichtung der Milch dem ersten Ventil nachfolgend angeordnet. Das vierte Ventil kann jedoch auch, wie oben bereits beschrieben, durch das erste Ventil gebildet sein. Ist es jedoch nachgeordnet, kann es beispielsweise als Flatterventil im Ventilkörper ausgebildet sein.

Vorzugsweise ist im Entleerungskanal ein fünftes Ventil vorhanden ist, welches sich nach Massgabe des angelegten Unterdrucks öffnet und schliesst. Dieses fünfte Ventil ist in Entleerungsrichtung der Milch dem zweiten Ventil nachfolgend angeordnet. Das fünfte

Ventil kann wie oben beschrieben durch das zweite Ventil gebildet sein. Ist es nachgeordnet, kann es beispielsweise ein Flatterventil im Ventilkörper sein.

Vorzugsweise ist mindestens ein Teil, vorzugsweise sind alle der genannten Ventile in einem gemeinsamen Ventilkörper angeordnet. Dies erleichtert die Herstellung und minimiert die Kosten.

Vorzugsweise ist der Ventilkörper einstückig und flexibel ausgebildet. Vorzugsweise besteht er aus Silikon.

Vorzugsweise weist die Brustpumpe ein Pumpengehäuse mit einem Antrieb für die Pumpmembran auf, wobei die steife erste Schale lösbar mit dem Pumpengehäuse verbunden ist. Dies erleichtert den Zusammenbau, auch nach einer Reinigung durch die Mutter.

Vorzugsweise ist die steife erste Schale Bestandteil eines des Hygienemoduls, welches gemeinsam mit dem Pumpengehäuse verbindbar ist, wobei das Hygienemodul des Weiteren mindestens die genannten Ventile aufweist. Dieses Hygienemodul ist vorzugsweise das oben genannte Hygienemodul.

Vorzugsweise ist die steife erste Schale Bestandteil eines Adapterteils, wobei der Ventilkörper in einer Aufnahme des Adapterteils gehalten ist. Dies erleichtert die Montage und ermöglicht insbesondere einen modulartigen Aufbau sowie die Verwendung eines Hygienemoduls.

Vorzugsweise umfasst das Hygienemodul ferner ein manuelles Betätigungsmittel zum zeitgleichen Betätigen des ersten und zweiten Ventils. Die manuelle Betätigung der zwei Ventile ist eine kostengünstige und betriebssichere Lösung.

Vorzugsweise sind das Adapterteil und das Betätigungsmittel nicht zerstörungsfrei voneinander lösbar. Dadurch ist sichergestellt, dass das Hygienemodul stets korrekt zusammengestellt und nur mit sämtlichen Elementen gemeinsam verwendet werden kann.

Vorzugsweise weist die Brustpumpe ein Pumpengehäuse auf, wobei die Brusthaube lösbar am Pumpengehäuse befestigbar ist und wobei durch die Befestigung der Brusthaube am Pumpengehäuse Teile, welche zwischen Brusthaube und Pumpengehäuse liegen, dichtend miteinander verbunden werden, so dass eine dichte Verbindung zwischen Pumpkammer und Innenraum der Brusthaube entsteht. Die Brusthaube lässt sich einfach mit der Hand halten, was den Zusammenbau der Pumpe erleichtert. Da die übrigen Teile durch die Befestigung der Brusthaube am Pumpengehäuse ebenfalls dicht anliegen, ist die Brustpumpe somit mit einem einzigen Handgriff, nämlich der Befestigung der Brusthaube am Pumpengehäuse, einsatzbereit. Ebenso einfach lassen sich die einzelnen Teile entfernen, um gereinigt zu werden. Vorzugsweise sind die Brusthaube und der Ventilkörper Teil des Hygienemoduls.

Vorzugsweise weist die Brustpumpe ein Pumpengehäuse auf, an welchem die Brusthaube lösbar befestigbar ist, wobei die Brustpumpe ferner einen Milchsammelbehälter aufweist, welcher am Pumpengehäuse mittels eines Schnappverschlusses befestigbar ist. Die Befestigung des Milchsammelbehälters mittels eines Schnappverschlusses ist unkonventionell. Üblicherweise werden Drehverschlüsse verwendet. Dank des Schnappverschlusses lässt sich der Behälter anders ausbilden und insbesondere schneller befestigen und entfernen.

Vorzugsweise weist der Milchsammelbehälter einen Grundkörper und einen Deckel auf, wobei der Deckel mindestens teilweise luftdurchlässig und flüssigkeitsundurchlässig ausgebildet ist. Dieser Deckel bildet somit ein Ventil, welches der Belüftung dient, welches aber auch ermöglicht, dass der Behälter ohne Flüssigkeitsverlust auf den Kopf gestellt werden kann. Vorzugsweise weist der Deckel eine luftdurchlässige und flüssigkeitsundurchlässige Membran auf. Vorzugsweise ist der Deckel zwischen dem Milchsammelbehälter und der Pumpe eingeklemmt und mittels Distanzstiften positionierbar.

Zudem ist der Deckel vorzugsweise mit einem Einwegventil versehen, welches von der Pumpkammer in den Milchsammelbehälter führt.

In einer bevorzugten Ausführungsform weist die Brustpumpe zum Abpumpen von menschlicher Muttermilch eine Brusthaube mit einem Innenraum zur Aufnahme einer Mutterbrust und eine Pumpkammer auf, wobei der Innenraum mittels der Pumpkammer mit einem sich zyklisch ändernden Unterdruck beaufschlagbar ist. Ein Milchkanal führt vom Innenraum durch eine erste Einlassöffnung als erste Durchgangsöffnung in die Pumpkammer, durch welchen abgepumpte Milch vom Innenraum der Brusthaube in die Pumpkammer fliesst. Die Pumpkammer weist eine Auslassöffnung auf, durch welche die abgepumpte Milch von der Pumpkammer in einen Milchsammelbehälter fliesst. Ein Entleerungskanal ist zwischen dem Innenraum der Brusthaube und der Pumpkammer vorhanden, welcher getrennt vom Milchkanal verläuft, wobei der Entleerungskanal mittels eines Ventils verschliessbar ist, wobei der Entleerungskanal während des Abpumpvorgangs geschlossen ist und am Ende des Abpumpvorgangs geöffnet ist. Dadurch lässt sich Milch am Ende des Abpumpvorgangs auf einfache Art und Weise aus der Brusthaube entfernen. Der Entleerungskanal wird vorzugsweise mittels der Pumpkammer mit einem Unterdruck beaufschlagt, um die Milch aktiv abzupumpen und in den Milchsammelbehälter zu drängen.

In einer bevorzugten Ausführungsform weist die Brustpumpe zum Abpumpen von menschlicher Muttermilch eine Brusthaube mit einem Innenraum zur Aufnahme einer Mutterbrust und eine Pumpkammer auf, wobei der Innenraum mittels der Pumpkammer mit einem sich zyklisch ändernden Unterdruck beaufschlagbar ist. Ein Milchkanal führt vom Innenraum durch eine erste Einlassöffnung als erste Durchgangsöffnung in die Pumpkammer, durch welchen abgepumpte Milch vom Innenraum der Brusthaube in die Pumpkammer fliesst. Die Pumpkammer weist eine Auslassöffnung auf, durch welche die abgepumpte Milch von der Pumpkammer in einen Milchsammelbehälter fliesst. Die Brustpumpe weist ein Pumpengehäuse, eine Pumpmembran und ein Hygienemodul auf, wobei das Hygienemodul mindestens eine steife erste Schale zur Bildung der Pumpkammer und die Brusthaube aufweist. Das Hygienemodul ist als Einheit am Pumpengehäuse befestigbar, wobei eine dichte Verbindung zwischen der Pumpkammer und dem Innenraum der Brusthaube geschaffen wird und wobei die Pumpmembran zwischen dem Hygienemodul und dem Pumpengehäuse dichtend gehalten ist. Dies erleichtert die Reinigung der relevanten Teile der Brustpumpe durch die Mutter und den Zusammenbau nach einer derartigen Reinigung.

Vorzugsweise weist das Hygienemodul ferner einen Ventilkörper auf, welcher zwischen Brusthaube und steifer ersten Schale angeordnet ist. Der Ventilkörper sowie die entsprechenden Bereiche des ersten und zweiten Teils, welche vorzugsweise Dichtsitze für die Ventile des Ventilkörpers aufweisen, bilden eine gemeinsame Ventileinheit, auch Ventilkonstrukt genannt.

Vorzugsweise weist das Hygienemodul ferner ein manuelles Betätigungsmittel zur Betätigung mindestens eines Ventils des Ventilkonstrukts auf.

In einer bevorzugten Ausführungsform weist die Brustpumpe zum Abpumpen von menschlicher Muttermilch eine Brusthaube zur Aufnahme einer Mutterbrust, ein Pumpengehäuse sowie einen Milchsammelbehälter auf, wobei das Pumpengehäuse einerseits lösbar mit der Brusthaube und andererseits lösbar mit dem Milchsammelbehälter verbindbar ist. Der Milchsammelbehälter ist mittels eines Schnappverschlusses am Pumpengehäuse befestigbar.

Vorzugsweise weist diese Brustpumpe eine Pumpkammer und einen Milchkanal auf, durch welchen abgepumpte Milch von einem Innenraum der Brusthaube in die Pumpkammer fliesst, wobei die Pumpkammer einen Ausgang aufweist, welcher mit dem Milchsammelbehälter verbunden ist und durch welchen die abgepumpte Milch in den Milchsammelbehälter fliesst.

In einer bevorzugten Ausführungsform ist ein Milchsammelbehälter zur Verbindung mit einem Pumpengehäuse einer Brustpumpe zum Abpumpen von menschlicher Muttermilch, vorhanden, wobei der Milchsammelbehälter einen Deckel aufweist, der dem Pumpengehäuse zugewandt ist und der mindestens in einem Bereich luftdurchlässig und flüssigkeitsundurchlässig ist.

In einer bevorzugten Ausführungsform weist die Brustpumpe zum Abpumpen von menschlicher Muttermilch eine Brusthaube zur Aufnahme einer Mutterbrust und ein Pumpengehäuse auf, wobei die Brusthaube lösbar mit dem Pumpengehäuse verbindbar ist. Die Brusthaube ist mittels eines Bajonettverschlusses mit dem Pumpengehäuse verbindbar.

In einer bevorzugten Ausführungsform ist eine Brusthaube vorhanden, welche zur Befestigung an einem Pumpengehäuse einer Brustpumpe dient. Die Brusthaube weist einen Innenraum zur Aufnahme der Mutterbrust und einen Stutzen zur Befestigung am Pumpengehäuse auf, wobei der Stutzen mit einem Bajonettverschluss versehen ist.

In einer bevorzugten Variante eines Verfahrens (nicht beansprucht) zum Betätigen einer Brustpumpe zum Abpumpen von menschlicher Muttermilch weist die Brustpumpe eine Brusthaube mit einem Innenraum zur Aufnahme einer Mutterbrust und eine Pumpkammer auf, wobei der Innenraum mittels der Pumpkammer mit einem sich zyklisch ändernden Unterdruck beaufschlagt wird. In einer ersten Pumpphase wird der Innenraum mit dem sich zyklisch ändernden Unterdruck beaufschlagt, um Milch aus der Mutterbrust abzupumpen, wobei der Innenraum mit abgepumpter Milch gefüllt wird, ohne dass abgepumpte Milch aus dem Innenraum hinausfliesst. In einer zweiten Pumpphase fliesst abgepumpte Milch aus dem Innenraum in die Pumpkammer und die Pumpkammer wird mit abgepumpter Milch gefüllt. In einer dritten Pumpphase fliesst die abgepumpte Milch von der Pumpkammer in einen Milchsammelbehälter, wobei der Innenraum und die Pumpkammer mit abgepumpter Milch gefüllt bleiben. Der Wechsel von der zweiten Pumpphase zur dritten Pumpphase erfolgt, indem ein Füllstand der abgepumpten Milch eine Auslassöffnung erreicht, welche von der Pumpkammer zum Milchsammelbehälter führt, wodurch Milch durch die Auslassöffnung von der Pumpkammer in den Milchsammelbehälter fliesst.

Dank dieses Verfahrens stellt das Pumpsystem relativ schnell von einem pneumatischen auf ein hydraulisches oder vorwiegend hydraulisches Pumpsystem um. Dadurch lassen sich kleinere Antriebe und leistungsschwächere Motoren verwenden.

Vorzugsweise ist die erste Pumpphase eine vollständig oder vorwiegend pneumatische Pumpphase und die zweite Pumpphase eine vollständig oder vorwiegende hydraulische Pumpphase.

In einer bevorzugten Variante des Verfahrens wird der Innenraum in der ersten Pumpphase annähernd vollständig gefüllt. In einer bevorzugten Variante werden der Innenraum und/oder die Pumpkammer in der zweiten und/oder dritten Pumpphase annähernd
vollständig mit abgepumpter Milch gefüllt.

Vorzugsweise erfolgt der Wechsel von der ersten Pumpphase zur zweiten Pumpphase, indem ein Füllstand der abgepumpten Milch eine erste Durchgangsöffnung erreicht, welche vom Innenraum zur Pumpkammer führt, wodurch Milch durch die erste Durchgangsöffnung vom Innenraum in die Pumpkammer fliesst.

Vorzugsweise fliesst in der dritten Pumpphase die abgepumpte Milch durch die erste Durchgangsöffnung in die Pumpkammer und durch die Auslassöffnung in den Milchsammelbehälter.

Vorzugsweise wird in einer vierten Pumpphase der Innenraum entleert und die darin befindliche, bereits abgepumpte Milch wird durch eine zweite Durchgangsöffnung aus dem Innenraum zum Milchsammelbehälter geführt.

Vorzugsweise ist die erste Durchgangsöffnung mit einem ersten Ventil verschliessbar und die zweite Durchgangsöffnung mit einem zweiten Ventil verschliessbar, wobei die zweite Durchgangsöffnung während der ersten bis dritten Pumpphase stets geschlossen ist.

Vorzugsweise ist die erste Durchgangsöffnung während der vierten Pumpphase stets geschlossen.

Vorzugweise führt die zweite Durchgangsöffnung vom Innenraum in die Pumpkammer und steht über die Pumpkammer in fluidkommunizierender Verbindung mit der Auslassöffnung.

Vorzugsweise wird während mindestens einem Teil der Pumpphasen, vorzugsweise während der ersten, zweiten und dritten Pumpphase ein Basisunterdruck beibehalten.

Vorzugsweise ist ein drittes Ventil vorhanden, welches bei Druckanstieg des sich zyklisch ändernden Unterdrucks in Richtung Atmosphärendruck zuerst öffnet und dann schliesst und dadurch den Basisunterdruck beibehält.

### KURZE BESCHREIBUNG DER ZEICHNUNGEN

Bevorzugte Ausführungsformen der Offenbarung werden im Folgenden anhand der Zeichnungen beschrieben, die lediglich zur Erläuterung dienen und nicht einschränkend auszulegen sind. In den Zeichnungen zeigen:
Figur 1 eine perspektivische Darstellung einer offenbarten Brustpumpe,
Figur 2 eine Explosionsdarstellung der Brustpumpe gemäss Figur 1 in einer perspektivischen Ansicht;
Figur 3 eine Explosionsdarstellung der Brustpumpe gemäss Figur 1 in einer weiteren perspektivischen Ansicht;
Figur 4 einen Teilschnitt durch die Brustpumpe gemäss Figur 1 in einer ersten perspektivischen Ansicht;
Figur 5 einen Teilschnitt durch die Brustpumpe gemäss Figur 1 in einer zweiten perspektivischen Ansicht;
Figur 6 einen Längsschnitt durch ein Hygienemodul, befestigt an einem Trägermodul der Brustpumpe gemäss Figur 1 in Explosionsdarstellung;
Figur 7 eine perspektivische Darstellung des zusammengesetzten Hygienemodul gemäss Figur 6;
Figur 8a eine perspektivische Darstellung einer Brusthaube der Brustpumpe gemäss Figur 1;
Figur 8b einen Teilschnitt durch die Brusthaube gemäss Figur 8a;
Figur 9a eine perspektivische Darstellung durch einen Ventilkörper der Brustpumpe gemäss Figur 1;
Figur 9b einen Teilschnitt durch den Ventilkörper gemäss Figur 9a;
Figur 9c einen Längsschnitt durch den Ventilkörper gemäss Figur 9a;
Figur 9d eine Ansicht des Ventilkörpers gemäss Figur 9a;
Figur 10a eine perspektivische Darstellung eines Stellrings der Brustpumpe gemäss Figur 1;
Figur 10b eine Ansicht des Stellrings gemäss Figur 10a;
Figur 11a eine perspektivische Darstellung eines Adapterteils der Brustpumpe gemäss

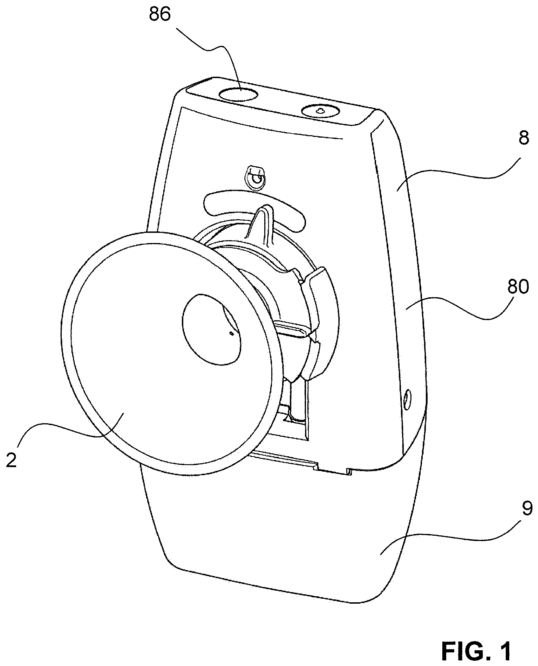

Figur 1 von einer ersten Seite;

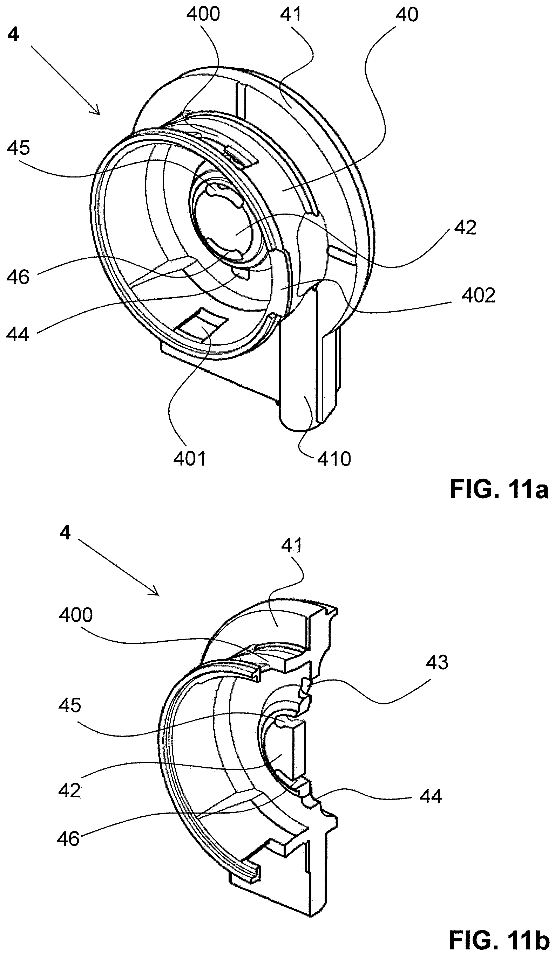

Figur 11b einen Teilschnitt durch das Adapterteil gemäss Figur I Ia;

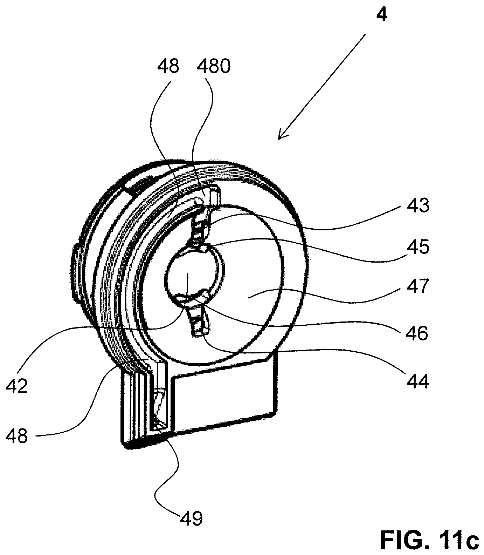

Figur 11c eine perspektivische Darstellung des Adapterteils gemäss Figur I Ia von einer zweiten Seite;

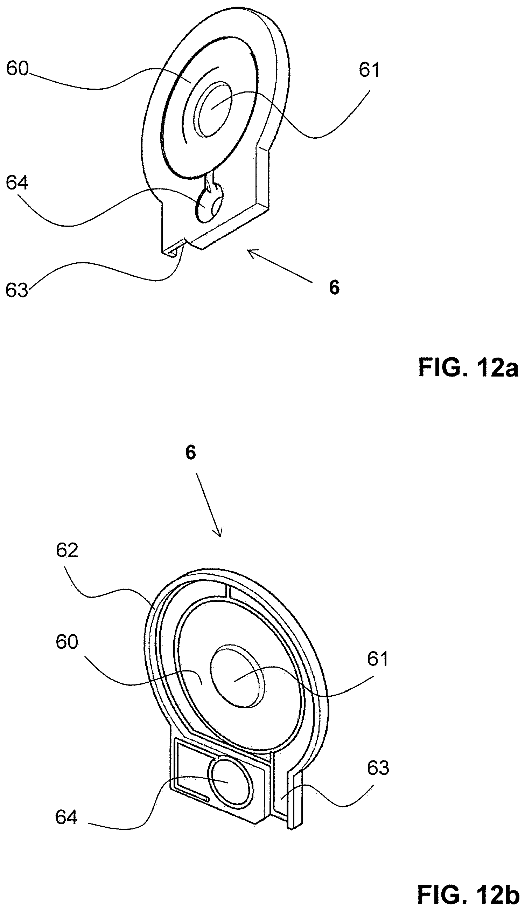

Figur 12a eine perspektivische Darstellung einer Medientrennmembran der Brustpumpe gemäss Figur 1 von einer ersten Seite;
Figur 12b eine perspektivische Darstellung der Medientrennmembran gemäss Figur 12a von einer zweiten Seite;

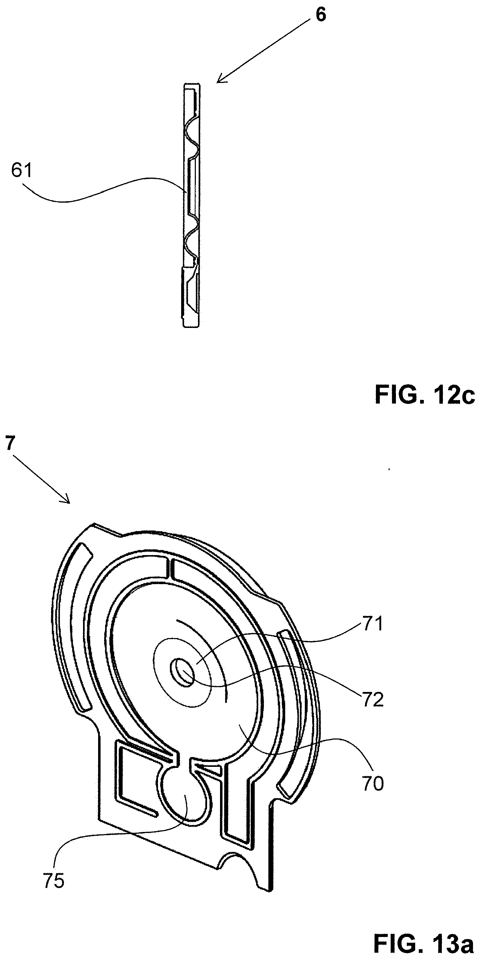

Figur 12c einen Längsschnitt durch die Medientrennmembran gemäss Figur 12a; Figur 13a eine perspektivische Darstellung einer Pumpmembran der Brustpumpe gemäss Figur 1 von einer ersten Seite;

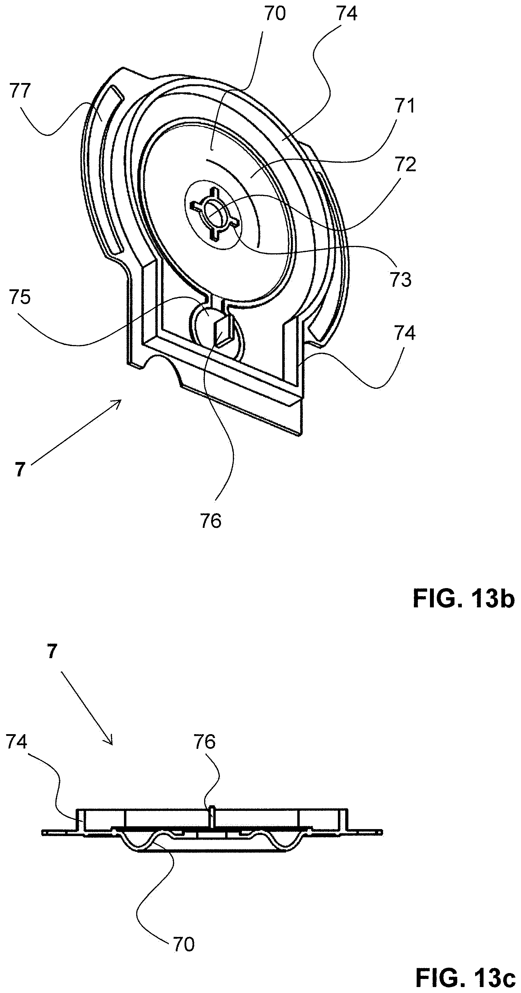

Figur 13b eine perspektivische Darstellung der Pumpmembran gemäss Figur 13a von einer zweiten Seite; Figur 13c einen Längsschnitt durch die Pumpmembran gemäss Figur 13a;

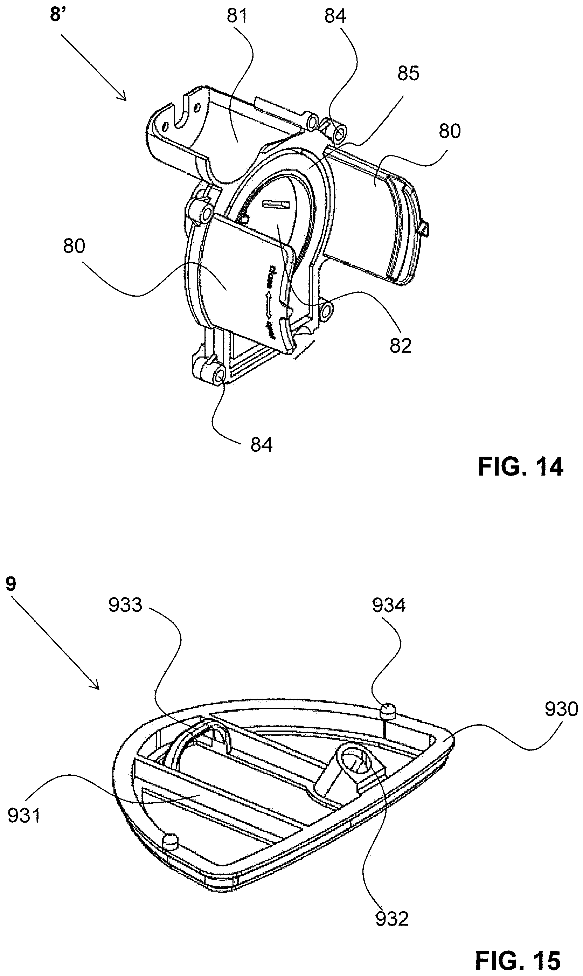

Figur 14 eine perspektivische Darstellung eines Trägermoduls der Brustpumpe gemäss Figur 1 ;
Figur 15 eine perspektivische Darstellung eines oberen Rahmenteils eines Milchsammelbehälters der Brustpumpe gemäss Figur 1;

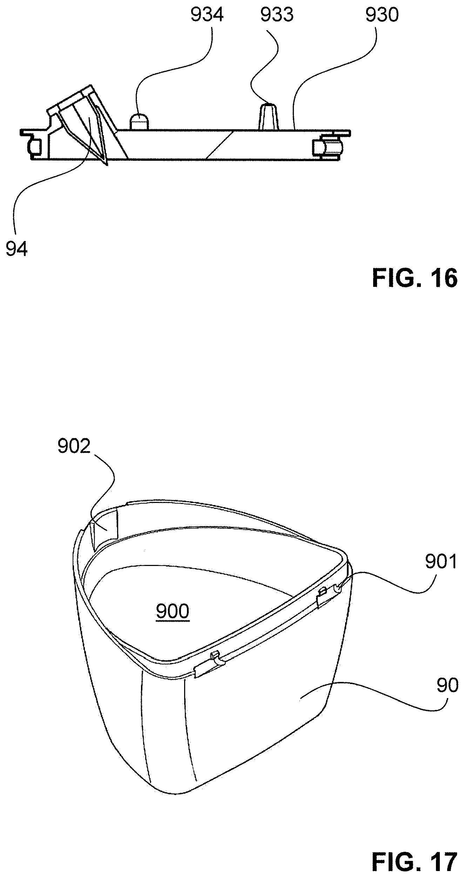

Figur 16 einen Längsschnitt durch das obere Rahmenteil gemäss Figur 15 mit einem Behälterventil;

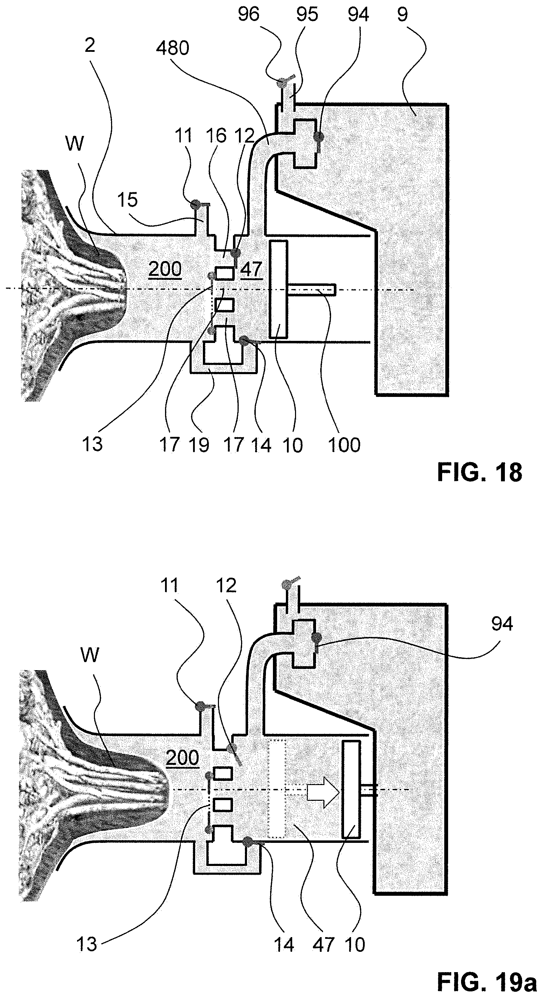

Figur 17 eine perspektivische Darstellung eines Gefässes des Milchsammelbehälters der Brustpumpe gemäss Figur 1 ; Figur 18 eine schematische Darstellung des erfmdungsgemässen Pumpsystems vor Anlegen eines Unterdrucks;
Figur 19a das schematisch dargestellte Pumpsystem gemäss Figur 1 bei der Aktivierung der Mutterbrust in einer ersten Hubposition;

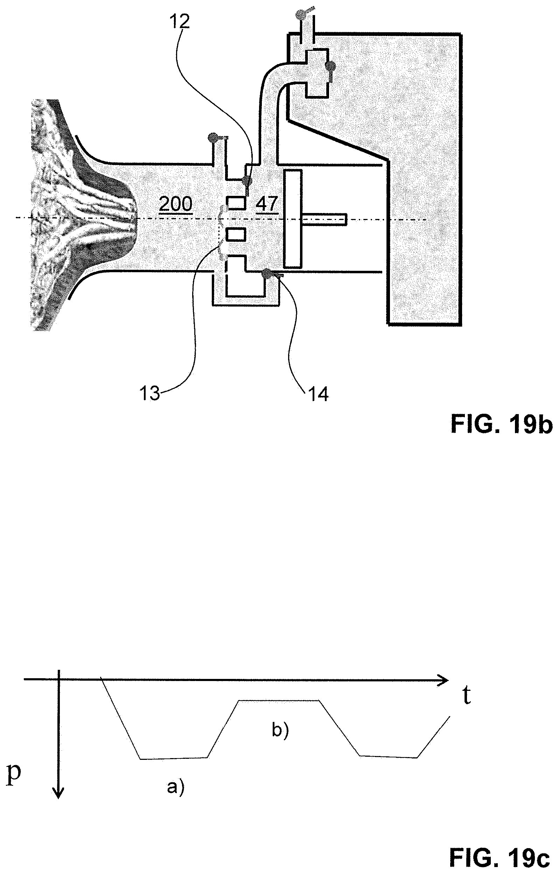

Figur 19b das Pumpsystem gemäss Figur 19a in einer zweiten Hubposition;

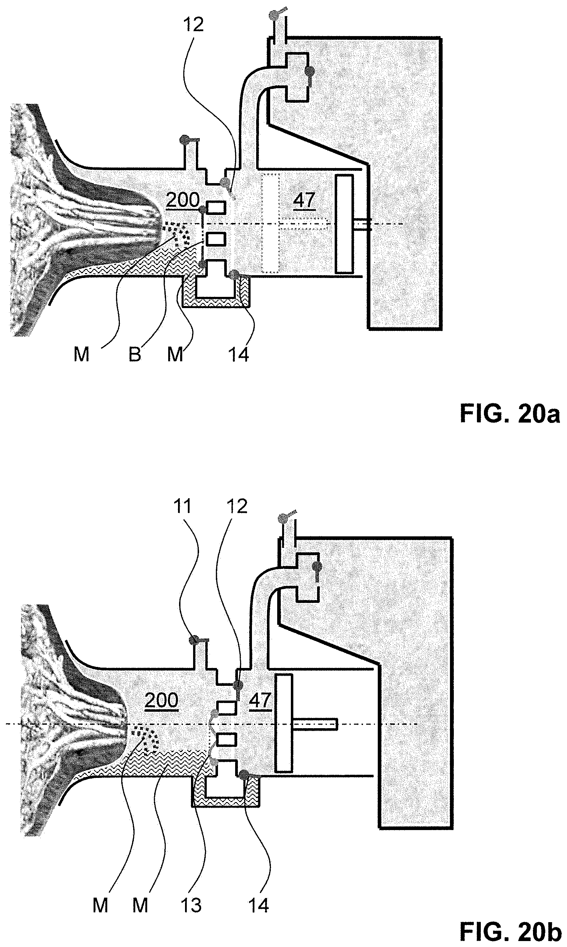

Figur 19c eine grafische Darstellung des Drucks innerhalb einer Brusthaube beim Wechsel von der ersten Hubposition in die zweite Hubposition gemäss den Figuren 19a und 19b; Figur 20a das Pumpsystem gemäss Figur 19a zu Beginn des Milchflusses aus der Brust in einer ersten Hubposition;
Figur 20b das Pumpsystem gemäss Figur 20a in einer zweiten Hubposition;

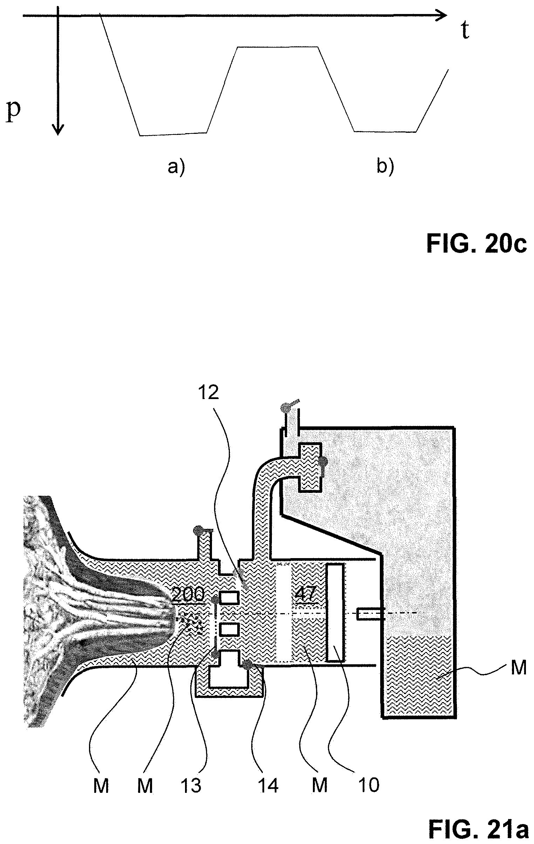

Figur 20c eine grafische Darstellung des Drucks innerhalb einer Brusthaube beim Wechsel von der ersten Hubposition in die zweite Hubposition gemäss den Figuren 20a und 20b;

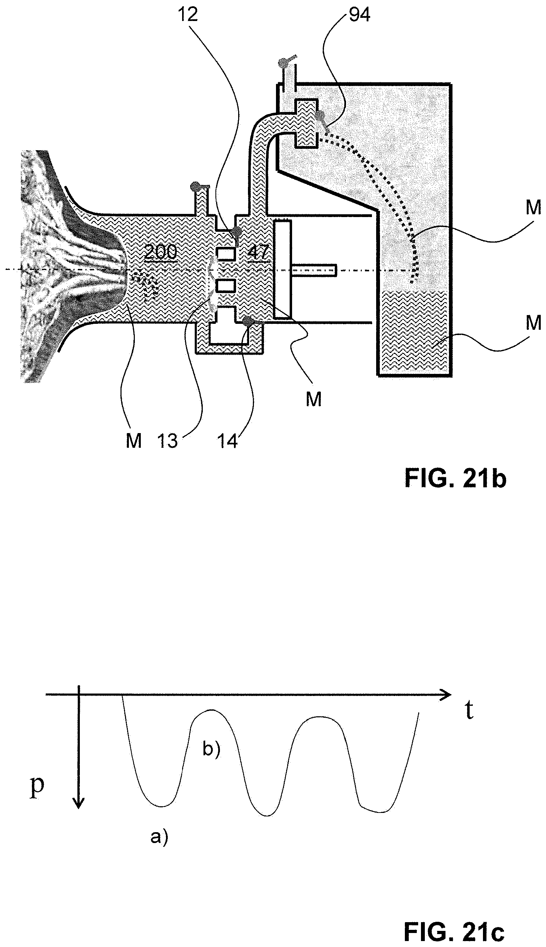

Figur 21a das Pumpsystem gemäss Figur 19a während des kontinuierlichen Milchflusses aus der Brust in einer ersten Hubposition; Figur 21b das Pumpsystem gemäss Figur 21 a in einer zweiten Hubposition;
Figur 21c eine grafische Darstellung des Drucks innerhalb einer Brusthaube beim Wechsel von der ersten Hubposition in die zweite Hubposition gemäss den Figuren 21a und 21b;

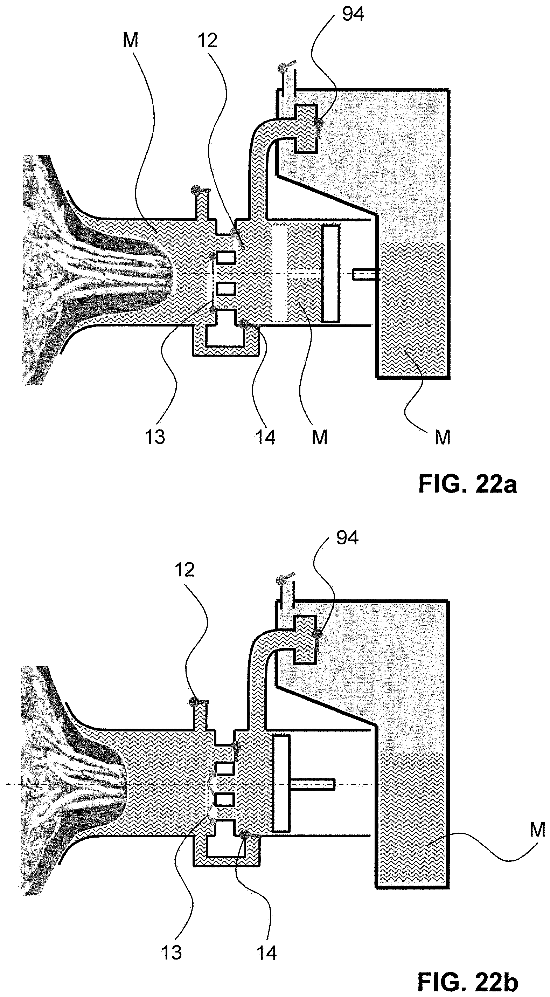

Figur 22a das Pumpsystem gemäss Figur 19a bei Beendigung des Milchflusses in einer ersten Hubposition;
Figur 22b das Pumpsystem gemäss Figur 22a in einer zweiten Hubposition;

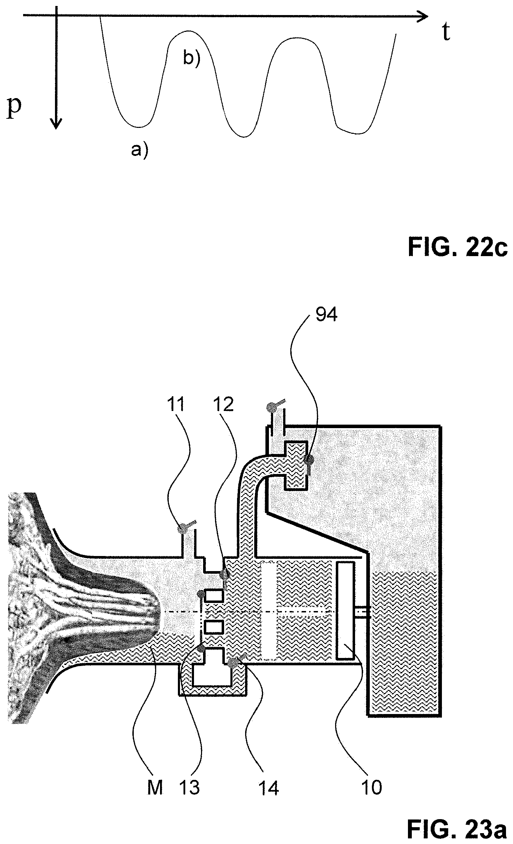

Figur 22c eine grafische Darstellung des Drucks innerhalb einer Brusthaube beim Wechsel von der ersten Hubposition in die zweite Hubposition gemäss den Figuren 22a und 22b;

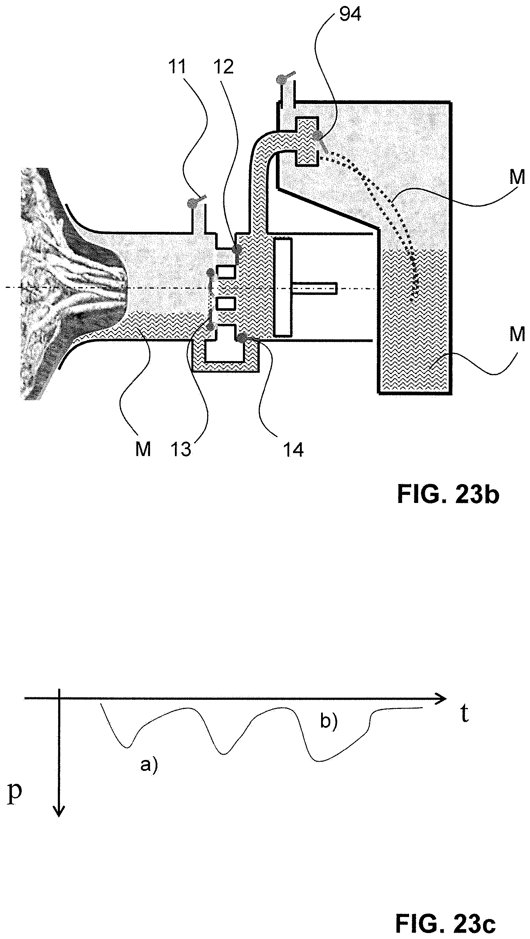

Figur 23 a das Pumpsystem gemäss Figur 19a beim Entleeren in einer ersten Hubposition;
Figur 23 b das Pumpsystem gemäss Figur 23 a in einer zweiten Hubposition und Figur 23c eine grafische Darstellung des Drucks innerhalb einer Brusthaube beim Wechsel von der ersten Hubposition in die zweite Hubposition gemäss den Figuren 23a und 23b.

### BESCHREIBUNG BEVORZUGTER AUSFÜHRUNGSFORMEN

In den Figuren 1 bis 17 ist ein bevorzugtes Ausführungsbeispiel der offenbarten Brustpumpe dargestellt.

Die Zusammenschau der einzelnen Bauteile

Wie in Figur 1 erkennbar ist, ist die Brustpumpe kompakt aufgebaut. Sie weist ein Pumpengehäuse 8 mit einer daran befestigten Brusthaube 2 und einem ebenfalls am Pumpengehäuse 8 befestigten Milchsammelbehälter 9 auf. Im Pumpengehäuse 8 ist ein Motor mit einem Kraftübertragungselement, beispielsweise eine Spindel, angeordnet. Im Pumpengehäuse 8 sind ferner mindestens eine elektronische Steuerung zur Steuerung des Motors, Betätigungsschalter 86 zur Betätigung der Pumpe und ein Energiespeicher vorhanden.

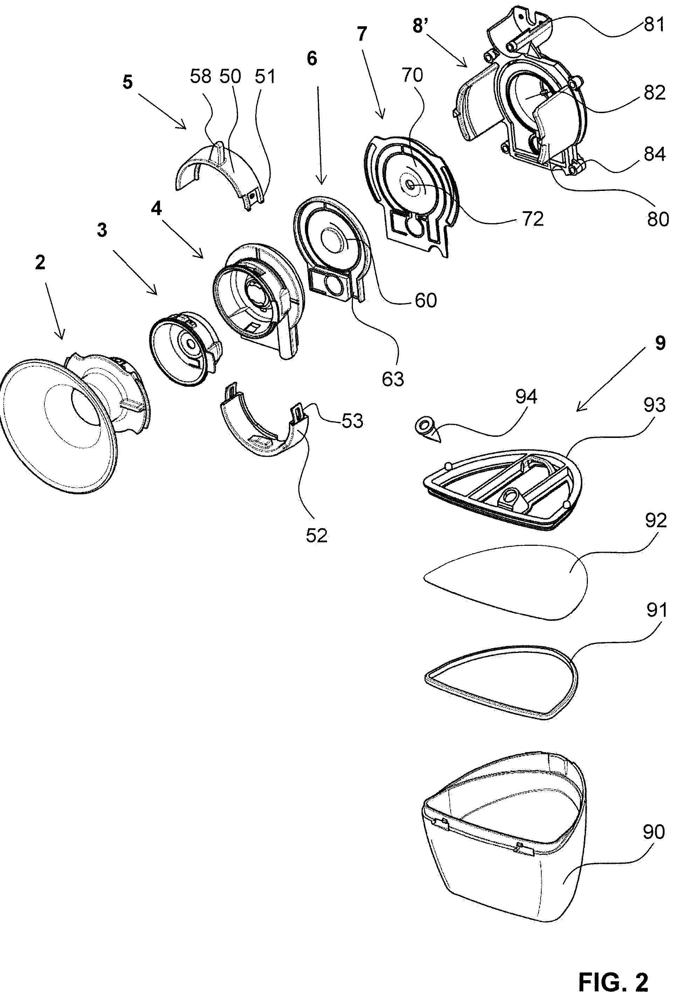

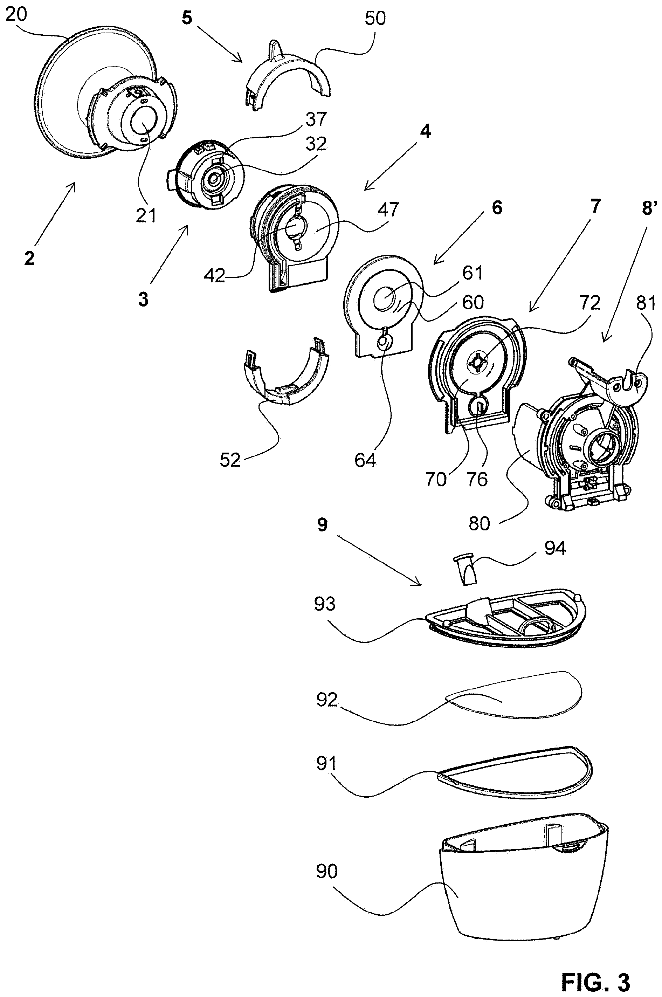

Die Figuren 2 und 3 zeigen eine Explosionsdarstellung dieser Brustpumpe. Sie umfasst die erwähnte Brusthaube 2 zur Aufnahme der Mutterbrust, einen Ventilkörper 3 aus einem flexiblen Material, insbesondere Silikon, ein Adapterteil 4, einen zweiteiligen Stellring 5, eine Medientrennmembran 6, eine Pumpmembran 7, ein Trägermodul 8', welches Teil des Pumpengehäuses 8 ist sowie den bereits erwähnten Milchsammelbehälter 9.

### Der Milchsammelbehälter

Der Milchsammelbehälter 9 ist vorzugsweise auf der Unterseite des Pumpengehäuses 8 an dieses einklinkbar, vorzugsweise lässt es einschwenkbar lösbar befestigen. Entsprechende Einrastelement 901, 902 sind in Figur 17 erkennbar. Das Pumpengehäuse 8 weist dazu passende, jedoch hier nicht dargestellte Einrastelemente auf.

Der Milchsammelbehälter 9 weist ein Gefäss 90 mit einem Innenraum 900 zur Aufnahme der abgepumpten Milch auf (siehe Figur 17). Dieses Gefäss 90 ist oben offen ausgebildet und mit einem Deckel verschliessbar. Der Deckel umfasst ein steifes unteres Rahmenteil 91, eine steife, halbsteife oder flexible Abdeckung 92 und ein steifes oberes Rahmenteil 93. Das untere Rahmenteil 91 entspricht der Form des Umfangs der Öffnung des Gefässes 90 und liegt dichtend auf diesem auf. Das steife obere Rahmenteil 93 klemmt die Abdeckung zwischen unterem und oberen Rahmenteil 91, 93 ein. Vorzugsweise sind die zwei Rahmenteile 91, 93 miteinander verleimt, verschweisst oder auf andere Art und Weise nicht zerstörungsfrei voneinander lösbar. Die Abdeckung 92 ist eine Folie oder ein anderes membranähnliches Teil, welche luftdurchlässig aber flüssigkeitsundurchlässig ist. Somit kann Luft aus dem Gefäss 90 entweichen, jedoch keine Flüssigkeit austreten.

Das obere Rahmenteil 93 entspricht vorzugsweise ebenfalls in seiner Form der Form des Gefässes 90, insbesondere entspricht sein Rahmen 930 der Form des unteren Rahmenteils 91. Es ist in den Figuren 15 und 16 detailliert dargestellt. Im Rahmen 930 sind zur Verstärkung und als Schutz der Abdeckung 92 Streben 931 vorhanden. Ein Griff 933 erleichtert das Anheben des Deckels. Distanzstifte 934, welche auf dem Rahmen 930 angeordnet sind und diesen überragen, gewährleisten bei montiertem Milchsammelbehälter 9 einen Abstand zum Pumpengehäuse 8, so dass Luft aus dem Behälterinnenraum 900 in die Umgebung entweichen kann. Eine Ventilaufnahme 932 ist am oberen Rahmenteil 93
angeformt und bildet den einzigen Flüssigkeitszugang in den Innenraum des Gefässes 90.

In dieser Ventilaufnahme 932 ist ein Behälterventil 94 angeordnet, welches als Einwegventil ausgebildet ist. Somit kann bei geschlossenem Deckel Flüssigkeit nur in den Milchsammelbehälter 9 hinein gelangen, nicht jedoch hinaus. Das Behälterventil 94 ist vorzugsweise, wie hier dargestellt, ein Entenschnabelventil.

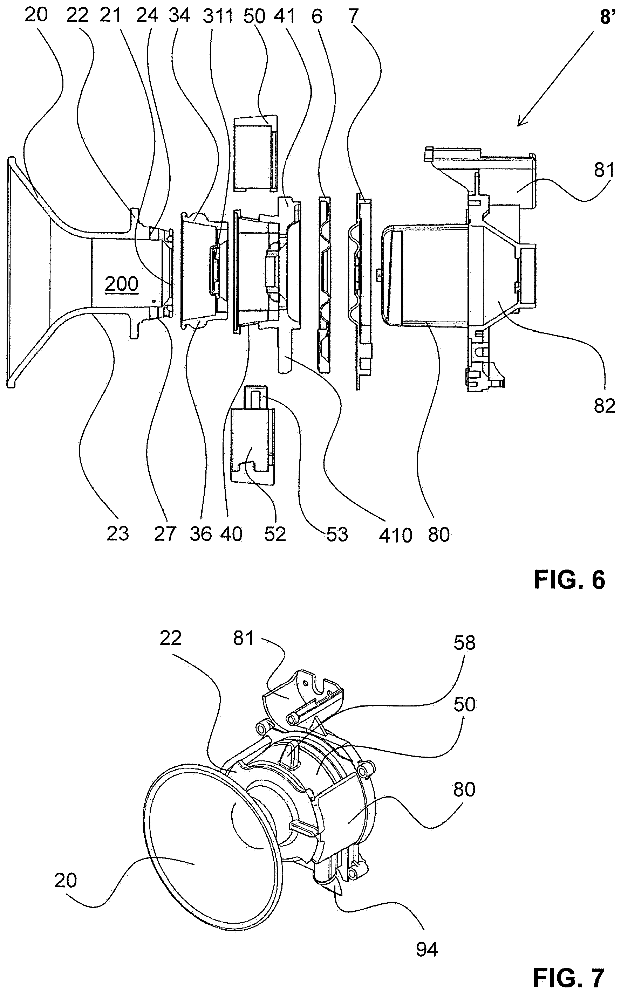

Das Hygienemodul In den Figuren 6 und 7 ist ein offenbartes Hygienemodul dargestellt. Es umfasst mindestens den Stutzen 23 der Brusthabe 2, den Ventilkörper 3, das Adapterteil 4 sowie den Stellring 5. Es kann zudem, wie hier dargestellt ist, auch eine flexible Medientrennmembran 6 umfassen. Ist die Brusthaube einstückig ausgeführt, so ist sie gesamthaft Teil des Hygienemoduls.

Die genannten Teile lassen sich, wie in den Figuren 6 und 7 dargestellt, zu einer gemeinsamen Baugruppe zusammenfügen und gemeinsam im Trägermodul 8' lösbar befestigen. Hierfür ist der Stutzen 23 der Brusthaube 2 vorzugsweise form- und kraftschlüssig in entsprechende Aufnahmen, hier zwei einander gegenüberliegende Seitenarme 80, des Trägermoduls 8' befestigbar. Vorzugsweise erfolgt die Verbindung über einen Bajonettverschluss, wie hier dargestellt ist. Der Stutzen 23 weist einen entsprechenden Flansch 22 auf, die Seitenarme 80 des Trägermoduls 8' entsprechende Aufnahmen für den Flansch und die übrigen Elemente des Bajonettverschlusses.

Dank der Befestigung der Brusthaube am Trägermodul 8' und somit am Pumpengehäuse 8 sind auch die dazwischenliegenden Teile, d.h. der Ventilkörper 3, das Adapterteil 4 und die Medientrennmembran 6 des Hygienemoduls sowie die Pumpmembran 7 miteinander dicht verbunden.

Der Stellring 5 ist vorzugsweise nicht zerstörungsfrei lösbar auf dem Adapterteil 4 befestigt, wobei er relativ zu diesem um die Längsmittelachse des Hygienemoduls schwenkbar ist.

Im Folgenden werden die einzelnen Teile der Vorrichtung und deren Zusammenwirken beschrieben.

### Die Brusthaube

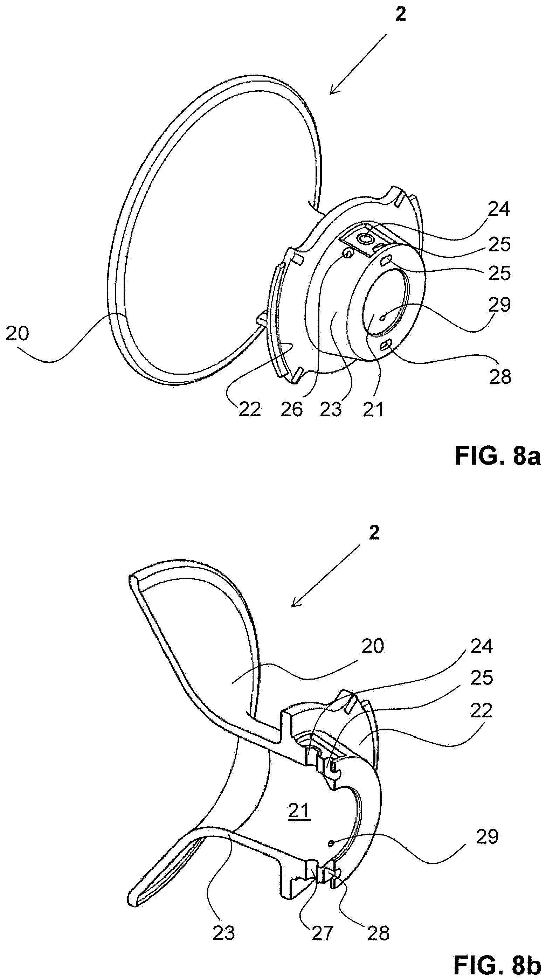

In den Figuren 8a und 8b ist die Brusthaube 2 dargestellt. Sie dient zur Aufnahme der Mutterbrust. Sie weist einen Trichter 20 zur Auflage auf die Mutterbrust und einen Innenraum 200 auf, welcher mit einem sich zyklisch ändernden Unterdruck beaufschlagbar ist und in welchem die Mutterbrust oder zumindest die Brustwarze aufgenommen wird. Die Brusthaube ist vorzugsweise einstückig und vorzugsweise aus einem steifen oder halbsteifen Kunststoff gefertigt. Sie kann jedoch auch mehrteilig ausgeführt sein. Sie kann zudem mit einem weichen Liner verwendet werden, z.B. mit einem Brusthaubeneinsatz aus einem weichen Silikon. Der Trichter 20 folgt ein Stutzen 23 mit einem mindestens teilweise umlaufenden Flansch 22, welcher einen Teil des genannten Bajonettverschlusses bildet. Der Innenraum 200 geht auf der brustabgewandten Seite des Stutzens 23 in eine Durchgangsöffnung 21 über, welche einen in der Mittellängsachse verlaufenden Kanal bildet. Am Stutzen 23, auf der brustabgewandten Seite des Flansches 22 ist, ist auf zwei diametral gegenüberliegenden Seiten je eine Vertiefung vorhanden.

In der ersten Vertiefung ist eine erste Auslassöffnung 24 sowie eine winkelförmige erste Durchflussöffnung 25 vorhanden. Die erste Auslassöffnung 24 führt vom Innenraum 200 zur Aussenseite des Stutzens 23, genauer in die erste Vertiefung. Die erste Durchflussöffnung 25 führt von dieser ersten Vertiefung in einem vorzugsweise rechtwinkligen Kanal zur brustabgewandten freien Stirnfläche des Stutzens 23. Auf dem Umfang des Stutzens 23 ist neben der ersten Auslassöffnung 24, aber jedoch ausserhalb der ersten Vertiefung, eine Belüftungsöffnung 26 vorhanden. Diese führt vom Innenraum 200 nach aussen.

Auf der diametral gegenüberliegenden Seite des Stutzens 23 ist eine analoge zweite Vertiefung mit einer zweiten Auslassöffnung 27 und einer zweiten Durchlassöffnung 28 vorhanden, welche gleich ausgebildet sind wie die ersten Öffnungen. Des Weiteren ist
ebenfalls ausserhalb der zweiten Vertiefung und neben der zweiten Auslassöffnung 27 eine zweite Belüftungsöffnung 29 vorhanden. Diese zweite Lüftungsöffnung 29 hat eigentlich keine Funktion, sondern ist bei bestimmungsgemässen Gebrauch der Vorrichtung verschlossen. Damit die Brusthaube 2 jedoch in um 180° gedrehter Position ebenfalls am Trägermodul 8' montiert werden kann, sind alle Öffnungen symmetrisch angeordnet.

### Der Ventilkörper

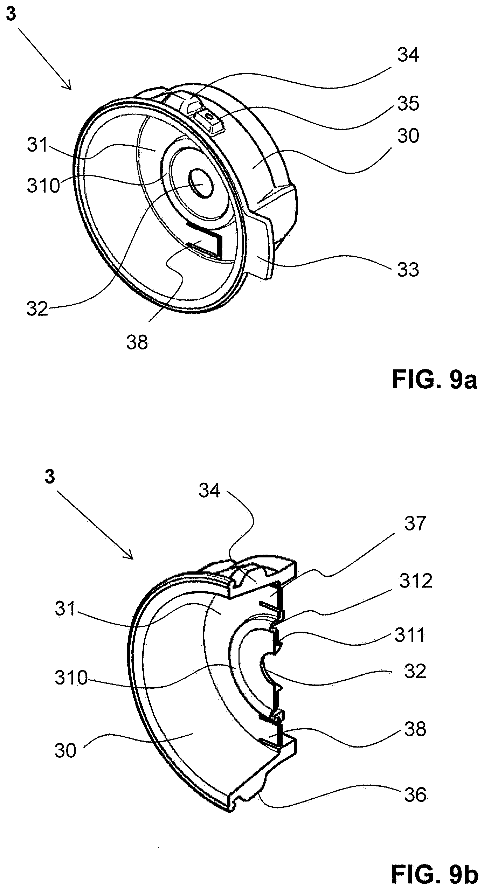

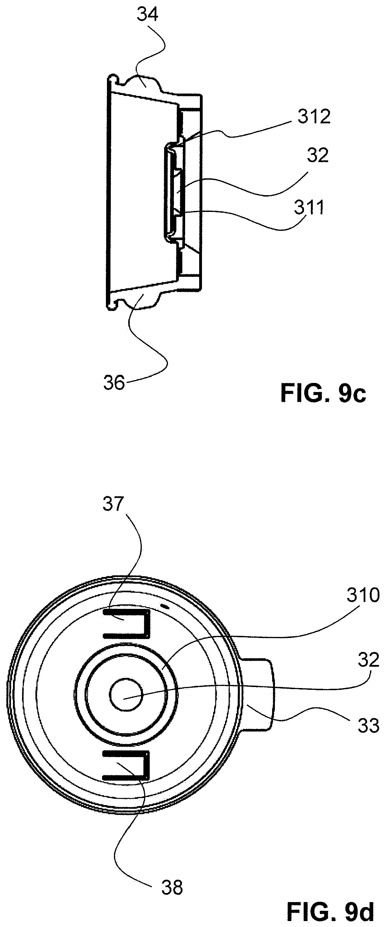

Die Figuren 9a bis 9d zeigen den Ventilkörper 3. Der Ventilkörper 3 ist aus einem flexiblen Material, vorzugsweise aus Silikon, gefertigt. Er ist vorzugsweise einstückig ausgebildet. Er weist einen hohlzylinderförmigen Grundkörper 30 mit einer Rückwand 31 auf. In der Rückwand befindet sich zentral eine Durchgangsöffnung 32. Die Rückwand 31 weist auf der brustabgewandten Seite eine die Durchgangsöffnung 32 ringförmig umlaufende Rille 312 auf, welche auf der gegenüberliegenden, brustnahen Seite eine Dichtfläche 310 bildet. Der umlaufende Rand der Durchgangsöffnung 32 ist auf der brustabgewandten Seite mit einer umlaufenden Dichtlippe 311 versehen (siehe Figuren 9b und 9c). Der Bereich der Rückwand 31 innerhalb der umlaufenden Dichtfläche 310 bildet somit ein Ventil, welches wie weiter unten erläutert ist, als BasisvakuumVentil dient.

Der Grundkörper 30 weist auf der brustzugewandten Seite eine radial abstehende Positioniernase 33 auf. Am Mantel des Grundkörpers 3 ist eine erste Noppe 34 sowie eine Belüftungsnoppe 35 vorhanden. Diametral gegenüberliegend befindet sich eine zweite Noppe 36. Die drei Noppen stehen radial nach aussen vor. Die erste Noppe 34 und die zweite Noppe 36 sind massiv, d.h. nicht hohl, ausgebildet. Die Belüftungsnoppe 35 weist eine Durchgangsöffnung vom Innenraum des Grundkörpers 30 nach aussen auf.

Im Ringbereich der Rückwand 31 zwischen Dichtungsfläche 310 und Mantel des Grundkörpers 30 sind einander ebenfalls diametral gegenüber liegende Ventilklappen vorhanden. Eine erste dieser Ventilklappen ist mit dem Bezugszeichen 37 versehen, eine zweite dieser Ventilklappen mit dem Bezugszeichen 38. Sie befinden sich vorzugsweise auf derselben Diagonalen wie die erste und der zweite Noppe 34, 36.

### Der Stellring

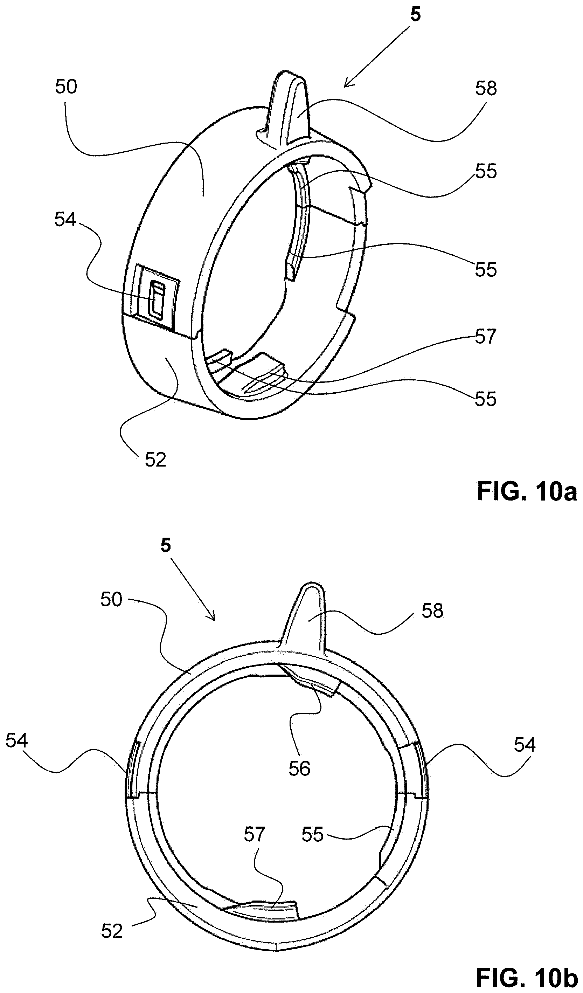

Die Figuren 10a und 10b zeigen den Stellring 5. Er ist vorzugsweise steif und
vorzugsweise aus Kunststoff ausgebildet. Der Stellring 5 besteht vorzugsweise aus zwei Teilen. Ein erstes Teil 50 weist die Form eines halben Ringes auf und ist an beiden freien Enden mit einem ersten Steckerteil 51 versehen. Der zweite Teil 52 weist ebenfalls die Form eines halben Ringes auf und ist an seinen freien Enden mit je einem zweiten Steckerteil 53 versehen. Der erste und zwei Teil 50, 52 bilden einen gemeinsamen Ring und die zwei Steckerteil-Paare 51, 53 sind mit Einrastelementen 54 versehen, um sich vorzugsweise unlösbar miteinander zu verbinden. Am inneren Umfang des Rings ist ein teilweise umlaufender, nach innen radial vorstehender Anschlag 55 vorhanden, welcher nach erfolgter Montage auf dem Adapterteil 4 ein erneutes Entfernen des Stellrings 5 verhindert. An der inneren Wandung des Stellrings ist ein erstes und ein zweites Anpresselement 56, 57 angeformt. Diese sind vorzugsweise radial nach innen vorstehende Erhöhungen, welche über eine Schrägfläche, auch Rampe genannt, in einen parallel zur Ringinnenfläche verlaufende Fläche übergehen. Am ersten Teil 50 ist ein nach aussen vorstehender Betätigungshebel 58 angeformt.

### Das Adapterteil

Die Figuren 11a bis 11c zeigen das Adapterteil 4. Es besteht vorzugsweise aus einem steifen Material, vorzugsweise aus Kunststoff. Es weist einen hohlzylinderförmigen Grundkörper 40 mit einer im Wesentlichen geschlossenen, flanschartigen pumpenseitigen Wand 41 auf. Der Grundkörper 40 weist in seinem Mantel ein erstes Fenster 400 und ein diametral gegenüberliegendes zweites Fenster 401 auf. An der brusthaubennahen Stirnfläche des Grundkörpers 40 ist eine Positionierhilfe 402 vorhanden, welche das Gegenstück zur Positioniemase 33 des Ventilkörpers 3 bildet und welche gemeinsam mit der Positioniernase 33 der drehsicheren Positionierung des Ventilkörpers 3 dient.

Die pumpenseitige Wand 40 verlängert sich in einer Richtung, welche hier als unten bezeichnet wird, in eine Kanalwand 410. Die pumpenseitige Wand weist ferner einen erhöhten zentralen Wandteil 42 auf, welcher in den brusthaubennahen Bereich ragt. Auf der gegenüberliegenden Seite des Wandteils 42, also dem Pumpengehäuse zugewandt, ist somit eine konkave Fläche ausgebildet, welche die bereits genannte steife erste Schale bildet und einen Teil der Pumpkammer 47 ist.

Zwischen dem erhöhten Wandteil 42 und Mantel des Grundkörpers 40 ist in der
pumpenseitigen Wand 41 eine erste Durchgangsöffnung 43 und eine zweite Durchgangsöffnung 44 vorhanden. Die zwei Durchgangsöffnungen 43, 44 liegen einander diametral gegenüber. Sie sind in diesem Beispiel kreisförmig ausgebildet. Im Randbereich des erhöhten Wandteils 42, jedoch bereits auf seiner Erhöhung, sind eine erste Rückspülöffnung 45 und eine zweite Rückspülöffnung 46 vorhanden. Diese sind vorzugsweise länglich ausgebildet. Diese Öffnungen führen alle in die Pumpkammer 47.

Wie in Figur 11c zu erkennen ist, ist die konkave steife erste Schale der Pumpkammer 47 von einem umlaufenden planen Rand umgeben, welche als Dichtfläche dient. In diesem Rand verläuft eine Rinne 48, welcher vorzugsweise an einem obersten Punkt der Pumpkammer 47 beginnt und mit dieser über eine Auslassöffnung 480 in fluidkommunizierender Verbindung steht. Der Kanal 48 verläuft entlang des Umfangs der Pumpkammer 47, hier teilkreisförmig, nach unten zu einem Auslass 49 hin. Der Auslass 49 befindet sich in der Kanalwand 410.

### Die Medientrennmembran

In den Figuren 12a bis 12c ist die Medientrennmembran 6 dargestellt. Sie ist flexibel ausgebildet, ist vorzugsweise einstückig und vorzugsweise aus Silikon gefertigt. Sie weist eine Membranfläche 60 auf, welche vorzugsweise in bekannter Weise mit mindestens einer ringförmigen Rille versehen ist. Ein Zentralbereich 61 der Membranfläche ist vorzugsweise erhöht ausgebildet und dem Adapterteil 4 zugewandt. Der Rand der Membranfläche 60 ist als Dichtfläche 62 ausgebildet und liegt dichtend am Rand des Adapterteils 4 an, wodurch die Pumpkammer 47 gebildet wird. Eine Rinne 63 verläuft innerhalb der Dichtfläche 62 entlang des Umfangs der Medientrennmembran 6 und bildet den dichtenden Verschluss der Rinne 48 des Adapterteils 4. Eine dem Pumpengehäuse zugewandte Vertiefung 64 im unteren Bereich der Medientrennmembran 6 dient als Freiraum für einen Drucksensor, welcher nachfolgend noch beschrieben ist.

Die Medientrennmembran 6 weist vorzugsweise, wie in den Figuren dargestellt ist, Versteifungen in Form von Rippen auf, welche die Funktionsweise der Medientrennmembran sicherstellen. D.h. die Rippen gewährleisten, dass sich die Membran in gewünschter Art und Weise bewegt und trotzdem stets dichtend an ihren benachbarten Teilen anliegt.

### Die Pumpmembran

In den Figuren 13a bis 13c ist die Pumpmembran 7 dargestellt. Auch sie weist eine Membranfläche 70 sowie einen Zentralbereich 71 auf. Im Gegensatz zur vollflächigen Medientrennmembran 6 ist der Zentralbereich 71 der Pumpmembran 7 mit einer Durchgangsöffnung 72 versehen, deren Randbereich vorzugsweise eine Verstärkung 73, hier in Form von Rippen, aufweist. Es ist wiederum eine umlaufende Dichtfläche 74 vorhanden, welche im zusammengesetzten Zustand der Vorrichtung dichtend auf der entsprechenden Fläche des Trägermoduls 8' anliegt. Ausserhalb der Dichtfläche 74 befinden sich Seitenflügel 77. Die Seitenflügel 77 bilden zusammen mit der umlaufenden planen Dichtfläche 74 (Figur 13b) im zusammengebauten Zustand eine dichte Barriere gegenüber von aussen einwirkenden Flüssigkeiten und schützen somit die Elektronik.

Der untere Bereich der Pumpmembran 7, welcher sich jedoch noch innerhalb der geschlossenen umlaufenden Dichtfläche 74 befindet, ist als Drucksensor ausgebildet. Der Drucksensor umfasst eine Sensorfläche 75 in Form einer Membran, welche über einen Verbindungssteg mit der Membranfläche 70 verbunden ist, und einer Fahne 76, welche der Sensorfläche senkrecht in Richtung Pumpengehäuse vorsteht. Diese Art Drucksensor ist in WO 2013/049944 A1 beschrieben.

Wie in den Figuren erkennbar ist, weisen die Medientrennmembran 6 und die Pumpmembran 7 ähnliche Formen auf, insbesondere bezüglich ihrer zur Erzeugung des Unterdrucks bewegbaren Bereiche.

### Das Trägermodul

Das Trägermodul 8' ist in Figur 14 dargestellt. Es ist in einem Aussengehäuse des Pumpengehäuses gehalten. Das Aussengehäuse ist in Figur 1 sichtbar. Vorzugsweise ist das Trägermodul 8' in diesem Aussengehäuse verschraubt. Die entsprechenden Befestigungslöcher sind mit dem Bezugszeichen 84 versehen. Das Trägermodul lässt sich einstückig aus Kunststoff herstellen. Es kann jedoch auch mehrteilig und/oder aus einem anderen Material gefertigt sein. Das Trägermodul 8' weist zwei vorstehende Seitenarme 10 mit einem Bajonettverschluss zur Aufnahme der Brusthaube 2 auf. Die Seitenarme sind seitlich an einer Spindelaufnahme 82 angeordnet. Die Spindelaufnahme 82 weist ein

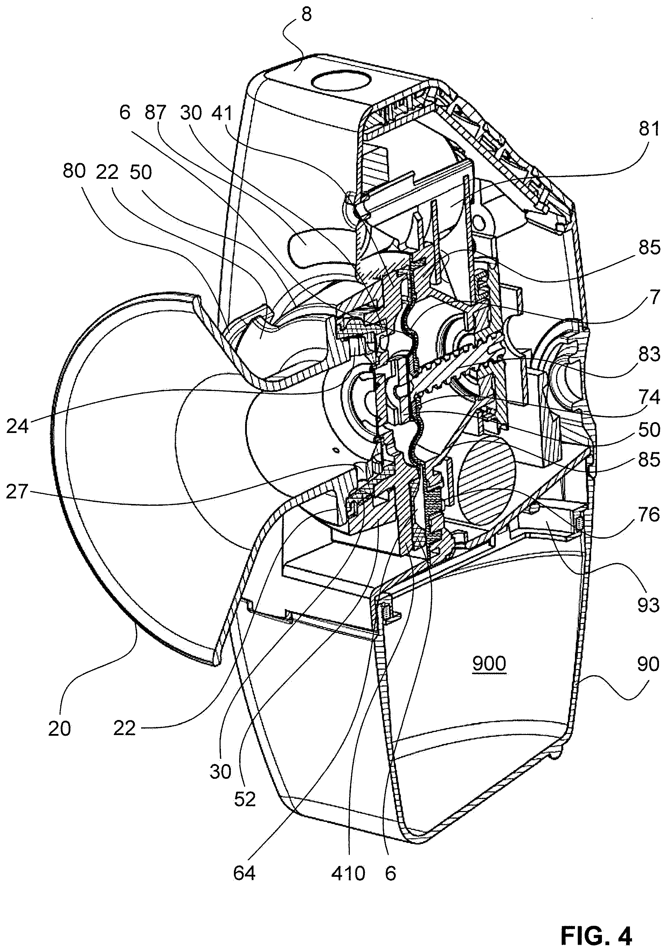

Durchgangsöffnung auf sowie einen umlaufenden Rand 85, welcher das Gegenstück zur Dichtfläche 74 der Pumpmembran 7 bildet. Auf der Spindelaufnahme 82 ist eine Motoraufnahme 81 in Form einer Schale ausgebildet. Die Motoraufnahme 81 dient der Halterung eines Elektromotors, welcher mit einer Spindel 83 (siehe Figur 4) verbunden ist.

### Wirkungsweise der zusammengesetzten Baugruppe

Anhand der Figur 6 lässt sich erkennen, wie die einzelnen Öffnungen und Ventile zusammenwirken.

Der Ventilkörper 3 lässt sich in den hohlzylindrischen Grundkörper 40 des Adapterteils 4 drehpositioniert einführen. Der Stellring 5 ist dabei bereits unlösbar mit dem Adapterteil 4 verbunden, wobei das erste Anpresselement 56 durch das erste Fenster 400 nach innen ragt und das zweite Anpresselement 57 durch das zweite Fenster 401 nach innen ragt. Die erste Noppe 34 des Ventilkörpers 3 befindet sich dabei unterhalb des ersten Fensters 400 und die zweite Noppe 36 oberhalb des ersten Fensters 401. Die Ventilklappen 37, 38 des Ventilkörpers 3 befinden sich jeweils über der ersten bzw. der zweiten Durchgangsöffnung 43, 44 des Adapterteils. Die Dichtlippe 31 1 liegt dichtend auf dem erhöhten Wandteil 42 auf, wobei die erste und zweite Rückspülöffnung in einem dichten Ringbereich zwischen Rille 312 und Dichtlippe 31 1 angeordnet sind.

Wird nun die Brusthaube 2 eingesetzt, so liegt die erste Noppe 34 über der ersten Auslassöffnung 24 und die Belüftungsnoppe 35 liegt über der ersten Belüftungsöffnung 26. Die zweite Noppe 36 liegt über der zweiten Auslassöffnung 27. Die zweite Belüftungsöffnung 29 ist vom Mantel des Ventilkörpers 4 überdeckt und somit verschlossen.

Wird nun die Brusthaube 2 mit dem Trägermodul 8' verbunden, so werden die einzelnen Verbindungen zwischen den beschriebenen Kanälen und Öffnungen erstellt und gegenüber aussen gedichtet.

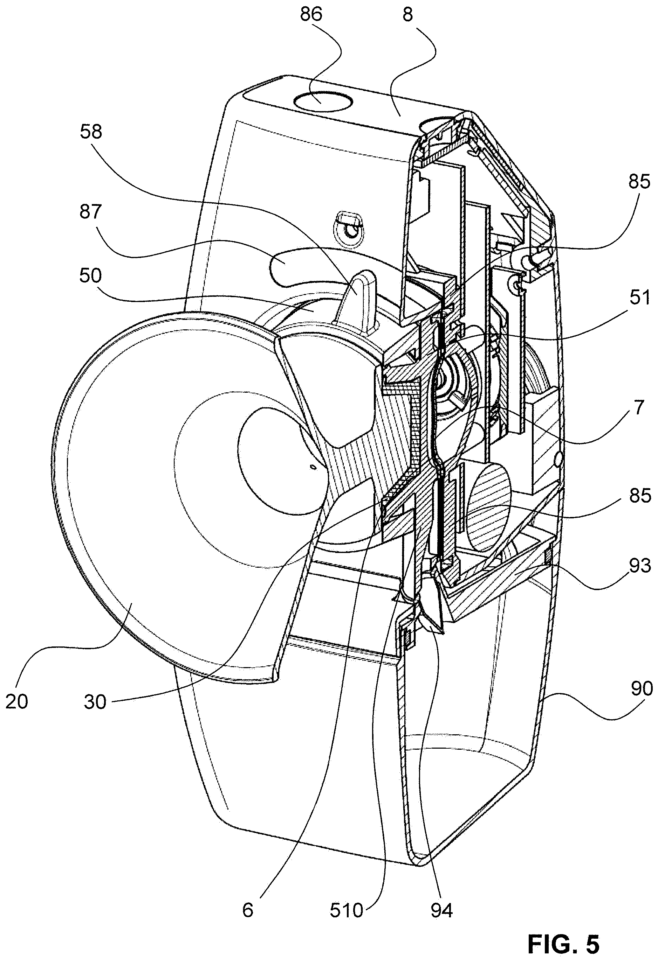

In den Figuren 4 und 5 ist die erfindungsgemässe Brustpumpe im zusammen gesetzten Zustand dargestellt. Wie gut erkennbar ist, weist dieses System praktisch keinen Totraum auf.

In Figur 4 ist die Spindel 83 erkennbar, welche die Pumpmembran 7 antreibt. Die Medientrennmembran 6 hat eine Form, welche sich der Pumpmembran 7 anschmiegt, so dass sich beide Membrane 6, 7 gemeinsam bewegen und auch hier keine Verluste auftreten. Das Pumpengehäuse 8, genauer das Trägermodul 8' weist eine Rückwand 85 auf, an welcher die Pumpmembran 7 dichtend anliegt, wenn das Hygienemodul am Trägermodul 8' befestigt ist. Dies ist in den Figuren 4 und 5 gut erkennbar. Der Stellring 5 ist schwenkbar innerhalb der Seitenarme 80 angeordnet, wobei der Betätigungshebel 58 auch als Anzeigeelement dient. Er bewegt sich entlang einer Anzeige 87, welche am Pumpengehäuse 8 angebracht ist und welche dem Benutzer somit Auskunft über die Stellung einzelner Ventile gibt und somit den Betriebszustand der Pumpe gibt. D.h. es wird angezeigt, ob die Pumpe betriebsbereit zum Abpumpen ist oder sich im Entleerungszustand befindet. Dies wird nachfolgend erläutert.

Durch Drehen des Stellrings 5 lassen sich einzelne Ventile manuell öffnen und schliessen. Genauer lassen sich die erste Auslassöffnung 24 gemeinsam mit der ersten Durchflussöffnung 25 alternativ zur zweiten Auslassöffnung 27 gemeinsam mit der zweiten Durchflussöffnung 28 manuell öffnen und schliessen. Die erste Belüftungsöffnung 26 wird gemeinsam mit der zweiten Auslassöffnung 27 und der zweiten Durchflussöffnung 28 geöffnet und geschlossen. Die zweite Belüftungsöffnung 29 bleibt aufgrund ihrer Position innerhalb des Ventilkörpers 3 stets geschlossen.

Ist die Brusthaube 2 derart relativ zum Ventilkörper 3 positioniert, dass die zweiten Öffnungen an der Position der ersten Öffnungen liegen, wird selbstverständlich die zweite anstelle der ersten Belüftungsöffnung geöffnet und geschlossen.

In einer ersten Stellung, die zu Beginn des Abpumpvorgangs und während des gesamten Abpumpvorgangs gewählt ist, wird die zweite Noppe 36 des Ventilkörpers 3 durch das zweite Anpresselement 57 des Stellrings nach unten gedrückt und liegt auf der Vertiefung und somit der zweiten Auslassöffnung 27 und der zweiten Durchflussöffnung 28 der Brusthaube 2 auf. Zudem wird die Belüftungsnoppe 35 durch das erste Anpresselement 56 auf die erste Belüftungsöffnung 26 gedrückt und verschliesst diese. Die erste Noppe 34 des Ventilkörpers 3 ist hingegen freigeben, so dass die erste Auslassöffnung 24 und die erste

Durchflussöffnung 25 einen fluidkommunizierenden Kanal vom Innenraum 200 der Brusthaube in den Grundkörper 30 des Ventilkörpers 3 bilden. Dies ist am besten in den Figuren 2, 3 und 6 erkennbar.

In einer zweiten Stellung wird der Stellring 5 gedreht. Das erste Anpresselement 56 gibt die Belüftungsnoppe 35 und somit die erste Belüftungsöffnung 26 frei, presst hingegen die erste Noppe 34 auf die Vertiefung und verschliesst somit die erste Auslassöffnung 24 und die erste Durchflussöffnung 25. Das zweite Anpresselement 57 gibt zudem die zweite Noppe 36 frei, so dass nun die zweite Auslassöffnung 27 und die zweite Durchflussöffnung 28 einen offenen, durchgehenden Kanal vom Innenraum 200 der Brusthaube 2 in den Ventilkörper 3 bilden. Die zweite Auslassöffnung 27 und die zweite Durchflussöffnung 28 bilden einen Entleerungskanal, welcher weiter unten noch genauer beschrieben wird.

Das gegenseitige Öffnen und Schliessen erfolgt vorzugsweise gleichzeitig. Es ist jedoch auch möglich, die Anpresselemente so versetzt anzuordnen, dass in einem Zwischenzustand alle Öffnungen verschlossen sind.

### Abpumpen der Muttermilch und Entleerungsvorgang

Damit Milch von der Mutterbrust abgepumpt werden kann, wird der Betätigungshebel 58 und somit der Stellring 5 in die erwähnte erste Stellung gebracht. Mittels des Antriebs wird die Pumpmembran 7 gemeinsam mit der Medientrennmembran 6 zum Adapterteil 4 hin und zurückbewegt, so dass in der Pumpkammer 47 und im Innenraum 200 der Brusthaube 2 ein Unterdruck entsteht. Die Vakuumleitung von der Pumpkammer 47 in den Innentraum 200 der Brusthaube 2 ist dabei gebildet durch die erste Durchgangsöffnung 43 im Adapterteil 4, die Öffnung im Ventilkörper 3 bedeckt durch die erste Ventilklappe 37 sowie den Kanal gebildet durch die erste Durchflussöffnung 25 und die erste Auslassöffnung 24 in der Brusthaube.

Das Basisvakuumventil, unter anderem gebildet durch die Dichtlippe 311 des Ventilkörpers 3, ermöglicht die Erhaltung eines Basisunterdrucks im Innenraum 200 der Brusthaube 2, wenn der Pumphub in Richtung Atmosphärendruck verläuft. Das Basisvakuumventil öffnet sich beim Pumphub in Richtung Atmosphärendruck und
schliesst erst bei Erreichen eines vorgegebenen Minimaldrucks im Innenraum 200. Die Wirkungsweise wird weiter unten anhand eines Schemas nochmals genauer erläutert. Der Rückfluss der Luft in die Brusthaube 2 erfolgt über die erste und zweite Rückspülöffnung 45, 46, des Adapterteils 4, die Durchgangsöffnung 32 des Ventilkörpers 3 und die Durchgangsöffnung 21 der Brusthaube 2.

Durch Anlegen dieses Vakuums an die Mutterbrust wird diese stimuliert und Milch fliesst aus der Brust in den Innenraum 200 der Brusthaube 2. Der Innenraum 200 füllt sich, bis der Füllstand der Milch die erste Auslassöffnung 24 erreicht. Die Brusthaube 2 wird dabei vorzugsweise so gehalten, dass sich diese erste Auslassöffnung 24 in einem oberen Bereich der Brusthaube 2 befindet.

Die abgepumpte Milch fliesst dann durch den Kanal, gebildet durch die erste Auslassöffnung 24 und die erste Durchlassöffnung 25, und durch die von der ersten Ventilklappe 37 bedeckten Öffnung in das Adapterteil 4, wo sie durch die erste Durchgangsöffnung 43 in die Pumpammer 47 gelangt.

Die Pumpkammer 47 füllt sich ebenfalls mit Milch, bis die oben angeordnete Auslassöffnung 480 erreicht ist. Die Milch fliesst anschliessend durch den Kanal, gebildet durch die Rinne 48 des Adapterteils 4 und der Rinne 63 bzw. der Abdeckung der Medientrennmembran 6 durch den Auslass 49 aus dem Hygienemodul heraus und durch das Behälterventil 94 in den Milchsammelbehälter 9.

Wenn der Pumphub jeweils von Erzeugung eines Unterdrucks in Richtung Atmosphärendruck, so öffnet sich zuerst das Basisvakuum- Ventil und ermöglicht einen Teilrückfluss der Luft bzw. der Milch Innenraum 200. Dieser Teilrückfluss erfolgt durch die erste und zweite Rückspülöffnung 45, 46, das offene Basisvakuumventil, die Durchgangsöffnung 32 und die Durchgangsöffnung 21. Das Ventil schliesst sich bei einem bestimmten Vakuumwert im Innenraum, so dass der Druck nicht weiter Richtung Atmosphärendruck ansteigen kann.

Wenn die Mutter das Abpumpen der Milch beenden will bzw. wenn keine Milch mehr aus der Brust fliesst, so schwenkt sie den Stellring 5 in die zweite Stellung. Der bisher benützte

Vakuum- und Milchkanal wird verschlossen, indem die erste Auslassöffnung 24 und die erste Durchlassöffnung 25 verschlossen werden. Dabei öffnen sich jedoch die zweite Auslassöffnung 27, die zweite Durchlassöffnung 28 und die erste Belüftungsöffnung 26. Diese Umstellung kann bei laufender Pumpe, d.h. bei nach wie vor angetriebener Pumpmembran 7, erfolgen. Die Pumpe kann hierfür auch abgestellt und anschliessend wieder in Betrieb genommen werden. Da die Pumpe nun weiter saugt, wird die sich noch im Innenraum 200 der Brusthaube 2 befindliche, bereits aus der Mutterbrust abgepumpte Milch durch die zweite Auslössöffnung 27, die zweite Durchlassöffnung 28, die durch die zweite Ventilklappe 37 verdeckte Öffnung des Ventilkörpers 3 und die zweite Durchgangsöffnung 44 des Adapterteils 4 in die Pumpkammer 47 gepumpt. Von dort wird sie durch die Bewegung der Pumpmembran 7 und der Medientrennmembran 6 aus der Pumpkammer 47 in Richtung Auslassöffnung 480 verdrängt. Sie fliesst somit ebenfalls durch die Rinne 48 und den Auslass 49 in den Milchsammelbehälter 9. Auf diese Weise lassen sich sowohl der Innenraum 200 der Brusthaube 2 wie auch die Pumpkammer 47 annähernd vollständig entleeren. Die Vorrichtung lässt sich zudem in eine Schräglage bringen, um die gesamte Milch aus dem System in den Milchsammelbehälter 9 zu entleeren.

Zu beachten ist, dass zu Beginn des Abpumpvorgangs die Brustpumpe vorzugsweise so gehalten wird, dass sich die erste Auslassöffnung 24 der Brusthaube und die Auslassöffnung 480 der Pumpkammer 47 in einem oberen Bereich des jeweiligen Hohlraums befinden. Dadurch kann Luft aus dem System entweichen und die zwei Hohlkammern werden relativ schnell mit Milch gefüllt. Dies ermöglicht ein möglichst rasches Wechseln von einem rein pneumatischen Pumpsystem mit Luft als Arbeitsfluid in ein reines oder mehrheitliches hydraulisches Pumpsystem, in welchem Milch das Arbeitsfluid bildet oder zumindest einen grossen Anteil des Arbeitsfluids bildet. Es ist jedoch nicht zwingend notwendig, dass die Öffnungen zu Beginn des Abpumpvorgangs derart angeordnet sind. Der Wechsel von einem Pumpsystem in das andere benötigt einfach längere Zeit und/oder kann nicht vollständig vollzogen werden.

Sobald die Milch den Innenraum 200 und die Pumpkammer 47 teilweise geflutet hat, vorzugsweise bis zu den genannten Öffnungen, kann die Brustpumpe in eine beliebige Position gebracht werden. Dies gilt nicht nur für die Brusthaube 2 und die Pumpkammer
47, sondern insbesondere auch für den Milchsammelbehälter 9. Dank des als Rückschlagventil ausgebildeten Behälterventils 94 und dank der flüssigkeitsundurchlässigen Abdeckung 92 kann der Milchsammelbehälter 9 sogar auf den Kopf gestellt werden, ohne den Betrieb der Brustpumpe zu beeinträchtigen.

In bevorzugten Ausführungsformen ist die Brusthaube und auch der Stellring beleuchtet, damit die Mutter auch in der Nacht während des Abpumpens eine Sichtkontrolle durchführen kann.

Die oben beschriebene Brustpumpe ist lediglich ein Ausftihrungsbeispiel, in welcher das offenbarte Prinzip umgesetzt und das Verfahren angewendet werden kann. In den Figuren 18 bis 23c ist deshalb nochmals schematisch dargestellt, worin der Kern der offenbarten Ideen liegt.

### Grundprinzip

In Figur 1 ist schematisch die Brusthaube 2 mit dem Innenraum 200 dargestellt. Die Brusthaube 2 ist dichtend auf eine Mutterbrust angelegt, die Brustwarze W ragt den Innenraum 200 hinein. Der Innenraum 200 ist über eine Auslassöffnung 16 mit einer Pumpkammer 47 verbunden. Eine zusätzliche Entleerungsöffnung 19 führt ebenfalls vom Innenraum 200 zur Pumpkammer 47. Die Auslassöffnung 16 ist mit einem Pumpventil 12 versehen, die Entleerungsöffnung mit einem Entleerungsventil 14. Ein Basisvakuumventil 13 schliesst eine dritte Öffnung 17 zwischen Pumpkammer 47 und Innenraum 200 der Brusthaube 2. Eine mit einem Belüftungsventil 11 verschliessbare Belüftungsöffnung 15 führt zudem vom Innenraum 200 der Brusthaube 2 nach aussen.

Das Volumen der Pumpkammer 47 ist mittels eines Pumpmittels 10, beispielsweise einer Pumpmembran oder einem Kolben, veränderbar. Das Pumpmittel 10 ist angetrieben, beispielsweise mittels einer Spindel 100.

Von der Pumpkammer 47 führt eine Auslassöffnung 480 in einen Milchsammelbehälter 9. Der Einlass in den Milchsammelbehälter 9 ist vorzugsweise mit einem Rückschlagventil, hier Behälterventil 94 genannt, versehen. Der Milchsammelbehälter 9 ist belüftet,
vorzugsweise ist hierfür ein Einlass 95 mit einem Einlassventil 96 vorhanden.

Der Vergleich mit dem oben ausführlich beschriebenen Ausführungsbeispiel zeigt, dass die Belüftungsöffnung 15 und das Belüftungsventil 11 der ersten Belüftungsöffnung 26, der Belüftungsnoppe 35 und dem ersten Anpresselement 56 entsprechen.

Die Auslassöffnung 16 und das Basisvakuumventil 12 der schematischen Darstellung entsprechen der ersten Auslassöffnung 24, der ersten Durchflussöffnung 25, der erste Noppe 34, dem ersten Anpresselement 56 sowie der ersten Ventilklappe 37 des konkreten Ausführungsbeispiels.

Die Entleerungsöffnung 19 und das Entleerungsventil 14 der schematischen Darstellung entsprechen der zweiten Auslassöffnung 27, der zweiten Durchflussöffnung 28, der zweiten Noppe 36, dem zweiten Anpresselement 57 sowie der zweiten Ventilklappe 38 des konkreten Ausführungsbeispiels.

Die Basisvakuumventil 13 sowie die dritte Öffnung 17 der schematischen Darstellung entsprechen der Dichtlippe 311, der ersten und zweiten Rückspülöffnung 45, 46 sowie der Durchgangsöffnung 32 im Ventilkörper 3 des konkreten Ausführungsbeispiels.

Das Behälterventil 94 der schematischen Darstellung entspricht dem Behälterventil des konkreten Ausführungsbeispiels.

Der Einlass 95 und das Einlassventil 96 der schematischen Darstellung sind in dem oben beschriebenen konkreten Ausführungsbeispiel durch die luftdurchlässige und flüssigkeitsundurchlässige Abdeckung, d.h. durch eine entsprechend gewählte Abdeckfolie, gebildet.

Diese Ventile lassen sich auch auf andere Art und Weise verwirklichen. Die Ventilöffnungen können auch in einem zusätzlichen Modulteil untergebracht sein, welches mit der Brusthaube lösbar verbunden ist. Zudem können die Auslassöffnung 16 und/oder die Entleerungsöffnung 19 als Kanal ausgebildet sein, welcher in einer Leitung, insbesondere einer flexiblen Leitung verläuft. Je nach Ausbildung dieser Leitung kann die Pumpkammer beabstandet zur Brusthaube und je nach Ausführungsform auch beabstandet zu einem Ventilmodul angeordnet sein. Die Ventile lassen sich vorzugsweise in einem Hygienemodul unterbringen, welches zwischen Pumpkammer und Brusthaubentrichter angeordnet ist. Ist die Pumpkammer beabstandet zur Brusthaube angeordnet, so kann das Ventilmodul benachbart zur Pumpkammer oder benachbart zur Brusthaube angeordnet sein bzw. ein Bestandteil der Brusthaube sein.

Im Folgenden wird die Funktionsweise erläutert:
In der Darstellung gemäss Figur 18 ist die Pumpe noch nicht eingeschaltet. Die Ventile sind bis auf das Einlassventil 96 des Milchsammelbehälters 9 geschlossen. In allen Bereichen herrscht Atmosphärendruck, d.h. p=0.

In der Darstellung gemäss den Figuren 19a bis 19c beginnt der Pumpvorgang und die Mutterbrust wird durch den angelegten Unterdruck stimuliert. Das Pumpventil 12 kann sich öffnen und schliessen. Im konkreten Ausführungsbeispiel ist hierfür die erste Auslassöffnung 24 und die erste Durchflussöffnung 25 freigegeben und die erste Ventilklappe 37 öffnet und schliesst das Ventil. Das Entleerungsventil 14 ist fest verschlossen. Im konkreten Ausführungsbeispiel erfolgt dies, indem der Stellring 5 in die entsprechende Position gebracht wird und die zweite Auslassöffnung 27 und die zweite Durchflussöffnung 28 fest verschliesst. Ebenso bleibt das Belüftungsventil 11 fest verschlossen. Im konkreten Ausführungsbeispiel wird dies erreicht, indem mittels des Stellrings 5 die erste Noppe 34 die erste Belüftungsöffnung 26 verschliesst.

Das Pumpmittel 10, vorzugsweise eine Pumpmembran oder ein Kolben, wird nach aussen gezogen, um einen

Unterdruck in der Pumpkammer 47 zu erzeugen (hier z.B. - 150 mmHg) (1 mmHg = 133.322 Pascals (Pa)). Das Pumpventil 12 öffnet sich und der Unterdruck wird in den Innenraum 200 der Brusthaube 2 übertragen, bzw. Luft strömt aus diesem Innenraum in die Pumpkammer 47. Auch im Innenraum herrscht somit ein Unterdruck, optimalerweise derselbe Druck von beispielsweise -150 mmHg. Der Druckverlauf im Innenraum 200 ist in Figur 19c dargestellt. Der Druck gemäss Bezugszeichen a) entspricht dem Druck im Innenraum 200 gemäss Figur 19a. Das Behälterventil 94 bleibt geschlossen. Der Druck im Milchsammelbehälter 9 beträgt nach wie vor Atmosphärendruck, d.h. p=0.

Wird das Pumpmittel 10 nun zurückgeschoben und die Pumpkammer 47 im Volumen verkleinert, so schliesst sich das Pumpventil 12 und der Druck in der Pumpkammer 47 erhöht sich bis zum Atmosphärendruck p-0. Der Druck im Innenraum 200 steigt aufgrund des nun geöffneten Basisvakuumventils 13 ebenfalls an. Da sich das Basisvakuumventil 13 jedoch bei einem bestimmten Unterdruck schliesst, wird ein Basisvakuum im Innenraum 200 beibehalten. Dieser liegt beispielsweise bei -20 mmHg. Diese Situation ist in Figur 19b gezeigt und der Druckverlauf in Figur 19c mit b) bezeichnet.

Durch Vergleich der Figuren 19a und 19b zeigt sich auch, wie sich die Brustwarze W im Innenraum 200 dehnt und wieder zusammenzieht.

In den Figuren 20a bis 20c hat der Milchfluss aus der Mutterbrust begonnen. Die Milch ist in den Figuren mit dem Bezugszeichen M versehen. Der Innenraum 200 der Brusthaube 2 füllt sich mit abgepumpter Milch. Es entsteht ein Milchsee unterhalb des Pumpventils 12. Dank der nun vorhandenen Milch erhöht sich die Pumpleistung bei gleichbleibender Antriebsleistung, beispielsweise auf -170 mmHg in der Pumpkammer 47 und dem Innenraum 200. Nach wie vor öffnet und schliessen sich bei jedem Pumphub das Pumpventil 12 und das Basisvakuumventil 13, wobei das Entleerungsventil 14 und das Belüftungsventil 1 1 geschlossen bleiben. Figur 20a zeigt die Situation bei maximalem Unterdruck (absoluter Wert), welcher auch mit a) in Figur 20c gekennzeichnet ist. Figur 20b zeigt die Situation bei Beibehaltung des Basisunterdrucks im Innenraum 200 und Atmosphärendruck in der Pumpkammer 47, was in Figur 20c mit b) gekennzeichnet ist.

Sobald sich der Innenraum 200 mit Milch M bis zur Auslassöffnungen 16 gefüllt hat, fliesst Milch M durch das Pumpventil 12 in die Pumpkammer 47. Die abgepumpte Milch M füllt nun auch die Pumpkammer 47 bis ihr Füllstand die Auslassöffnung 480 zum Milchsammelbehälter 9 erreicht. Nun öffnet sich das Behälterventil 94, vorzugsweise auf Grund des Drucks der angesammelten Milch M und Milch M fliesst in den Milchsammelbehälter 9.

Die Figuren 21a bis 21c zeigen die Situation, in welcher sich optimalerweise keine Luft mehr im System befindet und das ursprüngliche pneumatische System vollständig auf ein
hydraulisches System gewechselt hat. In der Praxis ist oft ein gemischtes System vorhanden, wobei die abgepumpte Milch vorzugsweise einen wesentlichen Teil der Volumina füllt und somit das Hauptarbeitsfluid bildet.

Wie bereits anhand der Figuren 19a bis 19c erläutert, öffnen und schliessen sich das Pumpventil 12 und das Basisvakuumventil 13 nach Massgabe des Pumphubs, so dass ein Unterdruck im Innenraum 200 erzeugt und eine Basisunterdruck beibehalten werden kann. Der Druck in der Pumpkammer 47 wechselt dabei beispielsweise zwischen -250 mmHg und 0 mmHg, der Druck im Innenraum zwischen -250 mmHg und -20 mmHg. Der Druck im Milchsammelbehälter 9 bleibt bei Atmosphärendruck, also bei 0 mmHg.

In den Figuren 22a bis 22c ist die Situation dargestellt, wenn keine Milch mehr aus der Brust fliesst. Das System pumpt zuerst noch weiter, wobei der Pumpdruck nicht mehr ausreicht, um das Behälterventil 94 zum Milchsammelbehälter 9 zu öffnen. Dies ist der Zeitpunkt, an welchem das Pumpventil 12 vollständig geschlossen und dafür das Entleerungsventil 14 aktiviert wird, so dass sich dieses nun selbsttätig öffnen und schliessen lässt. Im konkreten Ausführungsbeispiel wird hierfür der Stellring 5 manuell verstellt. Andere Arten, diese zwei Ventile zu aktivieren bzw. deaktivieren sind jedoch möglich. Beispielsweise lassen sie sich mit einer entsprechenden Elektronik steuern.

In den Figuren 23a bis 23c ist nun das Pumpventil 12 fest verschlossen und dafür der Entleerungskanal und das Entleerungsventil 14 geöffnet. Das Pumpmittel 10 bewegt sich weiter und erzeugt nach wie vom in der Pumpkammer 47 einen sich von einem maximalen Unterdruck zur Atmosphärendruck wechselnden Druck. Nach Massgabe dieser Bewegung öffnet und schliesst sich, wie vorher das Pumpventil 12, auch das Entleerungsventil 14 und pumpt somit die Milch M aus dem Innenraum 200 in die Pumpkammer 47. Von dort wird sie in den Milchsammelbehälter 9 gef rdert, vorzugsweise zuerst mittels Unterdruck und anschliessend mittels Verdrängung durch das Pumpmittel 10. Das Entleerungsventil 14 befindet sich in dieser Pumpsituation vorzugsweise an einer tiefen Stelle der Brusthaube, vorzugsweise relativ weit unten. Der sinkende Milchsee im Innenraum ist in den Figuren 23a und 23b gut erkennbar. Die Figuren 23a und 23b zeigen wiederum die zwei Extremsituationen eines Pumphubs. Der Druckverlauf ist in Figur 23c dargestellt. Der Druckverlauf im Innenraum 200 ist relativ unspezifisch.

Die Pumpe wird somit während des Entleerens weiterhin betätigt. Sollte sich zum Schluss noch Milch in der Pumpkammer 47 befinden, lässt sie sich durch Kippen oder "auf den Kopf stellen" der Pumpe einfach in den Milchsammelbehälter 9 leiten.

Die erfindungsgemässe Brustpumpe ermöglicht somit die Verwendung von abgepumpter Milch in einem hydraulischen Pumpsystem, wodurch der Antrieb der Pumpe minimiert werden kann.

**BEZUGSZEICHENLISTE**

| | | | |
|---|---|---|---|
| | | 32 | Durchgangsöffnung |
| 10 | Pumpmittel | 33 | Positioniernase |
| 100 | Spindel | 34 | erste Noppe |
| 11 | Belüftungsventil | 35 | Belüftungsnoppe |
| 12 | Pumpventil | 36 | zweite Noppe |
| 13 | Basisvakuumventil | 37 | erste Ventilklappe |
| 14 | Entleerungsventil | 38 | zweite Ventilklappe |
| 15 | Belüftungsöffnung | | |
| 16 | Auslassöffnung | 4 | Adapterteil |
| 17 | dritte Öffnung | 40 | Grundkörper |
| 19 | Entleerungsöffnung | 400 | erstes Fenster |
| | | 401 | zweites Fenster |
| 2 | Brusthaube | 402 | Positionierhilfe |
| 20 | Trichter | 41 | pumpenseitige Wand |
| 200 | Innenraum | 410 | Kanalwand |
| 21 | Durchgangsöffnung | 42 | erhöhter Wandteil |
| 22 | Flansch | 43 | erste Durchgangsöffnung |
| 23 | Stutzen | 44 | zweite Durchgangsöffnung |
| 24 | erste Auslassöffnung | 45 | erste Rückspülöffnung |
| 25 | erste Durchflussöffnung | 46 | zweite Rückspülöffnung |
| 26 | erste Belüftungsöffnung | 47 | Pumpkammer |
| 27 | zweite Auslassöffnung | 48 | Rinne |
| 28 | zweite Durchflussöffnung | 480 | Auslassöffnung |
| 29 | zweite Belüftungsöffnung | 49 | Auslass |
| 3 | Ventilkörper | 5 | Stellring |
| 30 | Grundkörper | 50 | erster Teil |
| 31 | Rückwand | 51 | erstes Steckerteil |
| 310 | Dichtungsfläche | 52 | zweiter Teil |
| 311 | Dichtlippe | 53 | zweites Steckerteil |
| 312 | Rille | 54 | Einrastelement |
| 55 | Anschlag | 83 | Spindel |
| 56 | erstes Anpresselement | 84 | Befestigungslöcher |
| 57 | zweites Anpresselement | 85 | Rand |
| 58 | Betätigungshebel | 86 | Betätigungsschalter |
| | | 87 | Anzeige |
| 6 | Medientrennmembran | | |
| 60 | Membranfläche | | |
| 61 | Zentralbereich | 9 | Milchsammelbehälter |
| 62 | Dichtfläche | 90 | Gefäss |
| 63 | Rinne | 900 | Innenraum |
| 64 | Vertiefung | 901 | erstes Einrastelement" |
| | | 902 | zweites Einrastelement |
| 7 | Pumpmembran | 91 | unterer Rahmenteil |
| 70 | Membranfläche | 92 | Abdeckung |
| 71 | Zentralbereich | 93 | oberer Rahmenteil |
| 72 | Durchgangsöffnung | 930 | Rahmen |
| 73 | Verstärkung | 931 | Streben |
| 74 | Dichtfläche | 932 | Ventilaufnahme |
| 75 | Sensorfläche | 933 | Griff |
| 76 | Fahne | 934 | Distanzstifte |
| 77 | Seitenflügel | 94 | Behälterventil |
| | | 95 | Einlass |
| 8 | Pumpengehäuse | 96 | Einlassventil |
| 8' | Trägermodul | | |
| 80 | Seitenarm | W | Brustwarze |
| 81 | Motoraufnahme | M | Milch |
| 82 | Spindelaufnahme | | |

## Patentansprüche

1. Brustpumpe zum Abpumpen von menschlicher Muttermilch, wobei die Brustpumpe eine Brusthaube (2) mit einem Innenraum (200) zur Aufnahme einer Mutterbrust und eine Pumpkammer (47) aufweist, wobei der Innenraum (200) mittels der Pumpkammer (47) mit einem sich zyklisch ändernden Unterdruck beaufschlagbar ist, wobei ein Milchkanal vom Innenraum (200) durch eine erste Einlassöffnung in die Pumpkammer (47) führt, durch welchen abgepumpte Milch vom Innenraum (200) der Brusthaube (2) in die Pumpkammer (47) fließt, und wobei die Pumpkammer (47) eine Auslassöffnung (480) aufweist, durch welche die abgepumpte Milch von der Pumpkammer (47) in einen Milchsammelbehälter (9) fließt, **dadurch gekennzeichnet, dass** die Milchpumpe derart ausgestaltet ist, dass in vertikaler Gebrauchslage der Brustpumpe mindestens zu Beginn eines Abpumpvorgangs die Auslassöffnung (480) in einem oberen Bereich der Pumpkammer (47) und/oder oberhalb der Einlassöffnung angeordnet ist, wobei die Pumpkammer (47) eine steife erste Schale und eine flexible Pumpmembran (7) aufweist, wobei die Pumpmembran (7) dichtend an der steifen ersten Schale anliegt und wobei die Pumpmembran (7) ausgebildet ist angetrieben zu werden und durch deren Bewegung relativ zur steifen ersten Schale ein Unterdruck in der Pumpkammer (47) erzeugbar ist.

2. Brustpumpe nach Anspruch 1, **dadurch gekennzeichnet, dass** der Milchkanal einen Vakuumkanal zur Beaufschlagung des Innenraums (200) der Brusthaube (2) mit dem sich zyklisch ändernden Unterdruck bildet.

3. Brustpumpe nach einem der vorangehenden Ansprüche, **dadurch gekennzeichnet, dass** zwischen der steifen ersten Schale und der flexiblen Pumpmembran (7) eine flexible Medientrennmembran (6) angeordnet ist, so dass die Pumpmembran (7) vor einem Kontakt mit abgepumpter Milch geschützt ist.

4. Brustpumpe nach einem der vorangehenden Ansprüche, **dadurch gekennzeichnet, dass** die erste Schale eine Auslassrinne aufweist, welche sich von der Auslassöffnung der Pumpkammer zu einem Ausgang erstreckt, wobei die Auslassrinne zumindest über einen Teilbereich in einer Richtung senkrecht zur Bewegungsrichtung der Pumpmembran verläuft, wobei bevorzugt die Pumpmembran (7) oder, falls vorhanden, die Medientrennmembran (6) die Auslassrinne der steifen ersten Schale zu einem bis auf die Auslassöffnung und den Ausgang geschlossenen Kanal verschließt.

5. Brustpumpe nach einem der vorherigen Ansprüche, **dadurch gekennzeichnet, dass** die Brusthaube (2) eine erste Durchlassöffnung (25) aufweist, welche Teil des Milchkanals bildet, wobei die Milchpumpe derart ausgestaltet ist, dass in vertikaler Gebrauchslage der Brustpumpe mindestens zu Beginn eines Abpumpvorgangs die Durchlassöffnung (25) in einem oberen Bereich des Innenraums (200) angeordnet ist, so dass der Innenraum (200) unterhalb der ersten Durchlassöffnung (25) mit abgepumpter Milch befüllbar ist.

6. Brustpumpe nach einem der vorherigen Ansprüche, **dadurch gekennzeichnet, dass** der Milchkanal mittels eines ersten Ventils verschließbar ist, um am Ende des Abpumpvorgangs die abgepumpte Milch aus der Pumpkammer (47) zu entfernen.

7. Brustpumpe nach einem der vorherigen Ansprüche, **dadurch gekennzeichnet, dass** ein Entleerungskanal zwischen dem Innenraum (200) der Brusthaube (2) und der Pumpkammer (47) vorhanden ist, welcher getrennt vom Milchkanal verläuft, und wobei der Entleerungskanal mittels eines zweiten Ventils verschließbar ist, wobei der Entleerungskanal während des Abpumpvorgangs geschlossen ist und am Ende des Abpumpvorgangs geöffnet ist.

8. Brustpumpe nach Anspruch 7, **dadurch gekennzeichnet, dass** ein Belüftungsventil (11) zur Belüftung des Innenraums (200) der Brusthaube (2) vorhanden ist, wobei vorzugsweise das Belüftungsventil (11) gemeinsam mit dem zweiten Ventil verschließbar ist und sich gemeinsam mit diesem öffnen lässt.

9. Brustpumpe nach Anspruch 7 oder 8, **dadurch gekennzeichnet, dass** ein drittes Ventil vorhanden ist, welches bei Druckanstieg des sich zyklisch ändernden Unterdrucks im Innenraum (200) der Brusthaube (2) einen Basisunterdruck beibehält, und/oder, dadurch dass im Milchkanal ein viertes Ventil vorhanden ist, welches sich nach Maßgabe des angelegten Unterdrucks öffnet und schließt und/oder dadurch dass im Entleerungskanal ein fünftes Ventil vorhanden ist, welches sich nach Maßgabe des angelegten Unterdrucks öffnet und schließt.

10. Brustpumpe nach einem der Ansprüche 6 bis 9, **dadurch gekennzeichnet, dass** mindestens ein Teil, vorzugsweise alle, der genannten Ventile in einem gemeinsamen Ventilkörper angeordnet sind.

11. Brustpumpe nach einem der vorangehenden Ansprüche, **dadurch gekennzeichnet, dass** die Brustpumpe ein Pumpengehäuse (8) mit einem Antrieb für die Pumpmembran (7) aufweist und wobei die steife erste Schale lösbar mit dem Pumpengehäuse (8) verbunden ist.

12. Brustpumpe nach Anspruch 11, **dadurch gekennzeichnet, dass** die steife erste Schale Bestandteil eines mehrteiligen Hygienemoduls ist, welches gemeinsam mit dem Pumpengehäuse (8) verbindbar ist.

13. Brustpumpe nach Anspruch 12, **dadurch gekennzeichnet, dass** das Hygienemodul ferner einen ersten Teil mit einer Durchflussöffnung zur Verbindung mit dem Innenraum (200), einen Ventilkörper (3) mit mindestens einem Ventil und einen zweiten Teil mit der steifen ersten Schale zur Bildung der Pumpkammer (47) aufweist, wobei der Ventilkörper (3) zwischen dem ersten und zweiten Teil angeordnet ist und wobei das Hygienemodul als Ganzes mit dem Pumpengehäuse (8) der Brustpumpe dichtend und lösbar verbindbar ist.

14. Brustpumpe nach Anspruch 11, **dadurch gekennzeichnet, dass** die steife erste Schale Bestandteil eines mehrteiligen Hygienemoduls ist, welches gemeinsam mit dem Pumpengehäuse (8) verbindbar ist, wobei die steife erste Schale Bestandteil eines Adapterteils (4) ist und wobei der Ventilkörper (3) in einer Aufnahme des Adapterteils (4) gehalten ist.

## Claims

1. A breast pump for pumping human breast milk, wherein the breast pump includes a breast shield (2) having an interior (200) for receiving a mother's breast and a pump chamber (47), wherein a cyclically changing negative pressure can be applied to the interior (200) by means of the pump chamber (47), wherein a milk channel leads from the interior (200) via a first inlet opening into the pump chamber (47) through which pumped milk flows from the interior (200) of the breast shield (2) into the pump chamber (47), and wherein the pump chamber (47) includes an outlet opening (480) through which the pumped milk flows from the pump chamber (47) into a milk collection container (9), **characterized in that** the milk pump is configured such that in a vertical usage orientation of the breast pump the outlet opening (480) is positioned in an upper region of the pump chamber (47) and/or above the inlet opening at least at the beginning of a pumping operation, wherein the pump chamber (47) includes a rigid first shell and a flexible pump membrane (7), wherein the pump membrane (7) sealingly abuts against the rigid first shell and the pump membrane (7) is configured such that it is driven and by the movement thereof relative to the rigid first shell generation of a negative pressure in the pump chamber (47) is enabled.

2. The breast pump according to claim 1, **characterized in that** the milk channel defines a vacuum channel for applying the cyclically changing negative pressure to the interior (200) of the breast shield (2).

3. The breast pump according to any one of the preceding claims, **characterized in that** between the rigid first shell and the flexible pump membrane (7) a flexible media separation diaphragm (6) is provided so that the pump membrane (7) is protected against contact with pumped milk.

4. The breast pump according to any one of the preceding claims, **characterized in that** the first shell includes an outlet groove extending from the outlet opening of the pump chamber to an output, wherein the outlet groove extends perpendicularly to the direction of movement of the pump membrane at least over a section, wherein preferably the pump membrane (7) or, if any, the media separation diaphragm (6) closes the outlet groove off the rigid first shell up to a channel closed except for the outlet opening and the output.

5. The breast pump according to any one of the preceding claims, **characterized in that** the breast shield (2) includes a first through opening (25) that is part of the milk channel, wherein the breast pump is configured such that in a vertical usage orientation of the breast pump the through opening (25) is positioned in an upper region of the interior (200) at least at the beginning of a pumping operation so that the interior (200) below the first through opening (25) can be filled with pumped milk.

6. The breast pump according to any one of the preceding claims, **characterized in that** the milk channel can be closed by means of a first valve for removing the pumped milk from the pump chamber (47) at the end of the pumping operation.

7. The breast pump according to any one of the preceding claims, **characterized in that** an evacuation channel is provided between the interior (200) of the breast shield (2) and the pump chamber (47) extending separately from the milk channel, and wherein the evacuation channel can be closed by means of a second valve, wherein the evacuation channel is closed during the pumping operation and opened at the end of the pumping operation.

8. The breast pump according to claim 7, **characterized in that** a vent valve (11) is provided for ventilation of the interior (200) of the breast shield (2), wherein preferably the vent valve (11) can concurrently be closed with the second valve and concurrently be closed with the same.

9. The breast pump according to claims 7 or 8, **characterized in that** a third valve is provided for keeping a baseline negative pressure when the cyclically changing negative pressure in the interior (200) of the breast field (2) increases and/or **in that** in the milk channel a fourth valve is provided which opens and closes according to the applied negative pressure and/or **in that** in the evacuation channel a fifth valve is provided which opens and closes according to the applied negative pressure.

10. The breast pump according to any one of claims 6 to 9, **characterized in that** at least part, preferably all, of the said valves are arranged in a common valve body.

11. The breast pump according to any one of the preceding claims, **characterized in that** the breast pump includes a pump housing (8) having a drive for the pump membrane (7), and wherein the rigid first shell is releasably connected to the pump housing (8).

12. The breast pump according to claim 11, **characterized in that** the rigid first shell is a component of a multipart hygiene module jointly connectable to the pump housing (8).

13. The breast pump according to claim 12, **characterized in that** the hygiene module further includes a first part including a flow-through opening for communication with the interior (200), a valve body (3) including at least one valve, and a second part including the rigid first shell for forming the pump chamber (47), wherein the valve body (3) is disposed between the first and the second parts and wherein the hygiene module as a whole can be sealingly and releasably connected to the pump housing (8) of the breast pump.

14. The breast pump according to claim 11, **characterized in that** the rigid first shell is a component of a multipart hygiene module jointly connectable to the pump housing (8), wherein the rigid first shell is a component of an adapter member (4) and wherein the valve body (3) is held in a receptacle of the adapter member (4).

## Revendications

1. Tire-lait destiné à extraire le lait maternel humain, le tire-lait comportant une téterelle (2) avec un espace intérieur (200) destiné à recevoir le sein maternel et une chambre de pompage (47), l'espace intérieur (200) pouvant être soumis à une dépression variant cycliquement au moyen de la chambre de pompage (47), un canal à lait menant de l'espace intérieur (200) à la chambre de pompage (47) à travers une première ouverture d'entrée, à travers laquelle le lait pompé s'écoule de l'espace intérieur (200) de la téterelle (2) dans la chambre de pompage (47), et la chambre de pompage (47) comportant une ouverture de sortie (480) à travers laquelle le lait pompé s'écoule de la chambre de pompage (47) dans un récipient de collecte de lait (9), **caractérisé en ce que** la pompe à lait est conçue de telle sorte que, dans la position d'utilisation verticale de la pompe à lait, au moins au début d'un processus de pompage, l'ouverture de sortie (480) est disposée dans une zone supérieure de la chambre de pompage (47) et/ou au-dessus de l'ouverture d'entrée, la chambre de pompage (47) comportant une première coque rigide et une membrane de pompage flexible (7), la membrane de pompage (7) reposant de manière étanche contre la première coque rigide et la membrane de pompage (7) étant conçue pour être entraînée et son mouvement par rapport à la première coque rigide permettant de créer une dépression dans la chambre de pompage (47).

2. Tire-lait selon la revendication 1, **caractérisé en ce que** le canal à lait forme un canal à vide pour alimenter l'espace intérieur (200) de la téterelle (2) avec la dépression qui varie cycliquement.

3. Tire-lait selon l'une des revendications précédentes, **caractérisé en ce qu'**une membrane flexible de séparation des fluides (6) est disposée entre la première coque rigide et la membrane de pompage flexible (7), de sorte que la membrane de pompage (7) est protégée de tout contact avec le lait pompé.

4. Tire-lait selon l'une des revendications précédentes, **caractérisé en ce que** la première coque comporte une goulotte d'évacuation qui s'étend de l'ouverture d'évacuation de la chambre de pompage vers une sortie, la goulotte d'évacuation s'étendant au moins sur une partie dans une direction perpendiculaire à la direction de mouvement de la membrane de pompage, la membrane de pompage (7) ou, le cas échéant, la membrane de séparation des fluides (6) ferme la goulotte d'évacuation de la première coque rigide pour former un canal fermé à l'exception de l'ouverture d'évacuation et de la sortie.

5. Tire-lait selon l'une des revendications précédentes, **caractérisé en ce que** la téterelle (2) présente une première ouverture de passage (25) qui fait partie du canal à lait, la pompe à lait étant conçue de telle sorte que, dans la position d'utilisation verticale de la pompe à lait, au moins au début d'un processus de pompage, l'ouverture de passage (25) est disposée dans une zone supérieure de l'espace intérieur (200), de sorte que l'espace intérieur (200) peut être rempli de lait pompé en dessous de la première ouverture de passage (25).

6. Tire-lait selon l'une des revendications précédentes, **caractérisé en ce que** le canal à lait peut être fermé au moyen d'une première valve afin d'évacuer le lait tiré de la chambre de pompage (47) à la fin du processus de pompage.

7. Tire-lait selon l'une des revendications précédentes, **caractérisé en ce qu**'un canal de vidange est prévu entre l'espace intérieur (200) de la téterelle (2) et la chambre de pompage (47), lequel s'étend séparément du canal à lait, et le canal de vidange pouvant être fermé au moyen d'une deuxième soupape, le canal de vidange étant fermé pendant le processus de pompage et ouvert à la fin du processus de pompage.

8. Tire-lait selon la revendication 7, **caractérisé en ce qu'**il est prévu une soupape d'aération (11) pour aérer l'espace intérieur (200) de la téterelle (2), la soupape d'aération (11) pouvant de préférence être fermée conjointement à la deuxième soupape et s'ouvrir conjointement à celle-ci.

9. Tire-lait selon la revendication 7 ou 8, **caractérisé en ce qu'**il est prévu une troisième soupape qui, en cas d'augmentation de la pression de la dépression variant cycliquement dans l'espace intérieur (200) de la téterelle (2), maintient une dépression de base, et/ou **en ce qu'**il existe une quatrième soupape dans le canal à lait, qui s'ouvre et se ferme en fonction de la dépression appliquée, et/ou **en ce qu'**il existe une cinquième soupape dans le canal de vidange, qui s'ouvre et se ferme en fonction de la dépression appliquée.

10. Tire-lait selon l'une des revendications 6 à 9, **caractérisé en ce qu'**au moins une partie, de préférence la totalité, des soupapes mentionnées sont disposées dans un corps de soupape commun.

11. Tire-lait selon l'une des revendications précédentes, **caractérisé en ce que** le tire-lait comprend un boîtier de pompe (8) avec un entraînement pour la membrane de pompage (7) et dans lequel la première coque rigide est reliée de manière amovible au boîtier de pompe (8).

12. Tire-lait selon la revendication 11, **caractérisé en ce que** la première coque rigide fait partie d'un module d'hygiène, comportant plusieurs parties, qui peut être relié au boîtier de pompe (8).

13. Tire-lait selon la revendication 12, **caractérisé en ce que** le module d'hygiène comprend en outre une première partie avec une ouverture d'écoulement pour la connexion à l'espace intérieur (200), un corps de soupape (3) avec au moins une soupape et une deuxième partie avec la première coque rigide pour former la chambre de pompage (47), le corps de soupape (3) étant disposé entre les première et deuxième parties, et le module d'hygiène pouvant être relié dans son ensemble de manière étanche et amovible au boîtier de pompage (8) de la pompe à lait.

14. Tire-lait selon la revendication 11, **caractérisé en ce que** la première coque rigide fait partie d'un module, comportant plusieurs parties, qui peut être relié au boîtier de pompe (8), la première coque rigide faisant partie d'une pièce d'adaptation (4) et le corps de soupape (3) étant maintenu dans un logement de la pièce d'adaptation (4).
